(19) Europäisches Patentamt
European Patent Office
Office européen des brevets

(11) **EP 3 928 324 B1**

(12) **FASCICULE DE BREVET EUROPEEN**

(45) Date de publication et mention de la délivrance du brevet:
**06.11.2024 Bulletin 2024/45**

(21) Numéro de dépôt: **20705372.9**

(22) Date de dépôt: **21.02.2020**

(51) Classification Internationale des Brevets (IPC):
***G16H 20/30*** *(2018.01)*     ***G06V 20/40*** *(2022.01)*

(52) Classification Coopérative des Brevets (CPC):
**G16H 20/30; G06V 20/42; G06V 20/53; G06V 40/23**

(86) Numéro de dépôt international:
**PCT/EP2020/054666**

(87) Numéro de publication internationale:
**WO 2020/169825 (27.08.2020 Gazette 2020/35)**

(54) **METHODE DE CARACTERISATION DE L'OCCUPATION DE L'ESPACE DANS LE DOMAINE DU SPORT PRENANT EN COMPTE LES DEPLACEMENTS DES JOUEURS ET DE LA BALLE**

VERFAHREN ZUR CHARAKTERISIERUNG DER RAUMBELEGUNG IM BEREICH DES SPORTS UNTER BERÜCKSICHTIGUNG DER BEWEGUNGEN DER SPIELER UND DES BALLES

METHOD FOR CHARACTERIZING THE OCCUPATION OF SPACE IN THE FIELD OF SPORT TAKING INTO ACCOUNT THE MOVEMENTS OF THE PLAYERS AND THE BALL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.02.2019 FR 1901772**

(43) Date de publication de la demande:
**29.12.2021 Bulletin 2021/52**

(73) Titulaires:
• **Ecole Polytechnique**
  **91128 Palaiseau Cedex (FR)**
• **Centre National de la Recherche Scientifique (C.N.R.S.)**
  **75794 Paris (FR)**

(72) Inventeurs:
• **BACOT, Vincent**
  **92130 Issy-Les-Moulineaux (FR)**
• **CLANET, Christophe**
  **91120 Palaiseau (FR)**

(74) Mandataire: **Hautier IP**
  **20, rue de la Liberté**
  **06000 Nice (FR)**

(56) Documents cités:
**KR-A- 20180 063 777     US-A1- 2014 244 007**

**Description**

**DOMAINE TECHNIQUE DE L'INVENTION**

Le domaine de l'invention est celui du sport.

Plus précisément, l'invention concerne un procédé d'analyse d'une occupation dynamique d'un terrain par deux équipes adversaires d'un jeu et un système associé.

L'invention trouve notamment des applications dans le domaine du sport collectif tel que le football, le rugby, le handball, le basketball, le hockey, le baseball, le volleyball ou le football américain. L'invention trouve également des applications dans le domaine des sports de raquettes, tels que le tennis, le badminton ou le squash.

**ÉTAT DE LA TECHNIQUE**

Il est connu de l'art antérieur des techniques d'analyse et de restitution des positions des joueurs sur un terrain afin de permettre à un entraineur d'analyse une phase de jeu, également appelée séquence de jeu, et d'améliorer la tactique appliquée par les joueurs d'une équipe.

Il existe par exemple des techniques permettant de fournir des statistiques sur les phases de jeu, telles que le nombre de passes réalisées d'une équipe, en analysant les positions des joueurs. Une telle technique est par exemple décrite dans le brevet américain US 8,279,051, mais est restreinte à une analyse statistique du jeu de chaque joueur, sans tenir compte de l'occupation territoriale des deux équipes.

Afin de pouvoir analyse des phases de jeu, il a été développé des techniques telles que celle décrite dans la demande internationale de brevet publiée sous le numéro WO2017/106390 où une séquence de jeu est associée par des méthodes de regroupement statistiques à des séquences de jeu similaires d'autres parties, stockées dans une base de donnée. Avec cette technique, il est notamment possible d'obtenir une prédiction sur l'évolution d'une séquence de jeu. Par exemple, dans le cadre d'un match de football, il peut ainsi être indiqué une probabilité de marquer au but en fonction de la position du joueur ayant le ballon par rapport au but et par rapport à la position des autres joueurs, notamment des joueurs de l'équipe adverse, au regard des séquences de jeu similaires d'autres parties.

Toutefois, cette technique étant basée sur une analyse statistique des phases de jeu précédentes présente l'inconvénient de permettre qu'une analyse limitée des phases de jeu car il est difficile de caractériser la performance d'un joueur ou d'une équipe, et de prévoir avec une telle technique des phases de jeu avantageuses qui n'auraient pas été jouées précédemment.

Malgré ces développements, il existe toujours un besoin d'information additionnelle sur la tactique de jeu, notamment pour caractériser l'occupation de l'espace par chaque équipe.

Ainsi, des techniques d'analyse permettant de partitionner une image d'un terrain de jeu pour analyser l'occupation du terrain par chaque équipe ont été développées.

Une telle technique est par exemple décrite dans la demande internationale de brevet WO03/037464 où un terrain de football est partitionné en utilisant des diagrammes de Voronoï. Cette partition du terrain est effectuée en considérant soit une des deux équipes, avec ou sans le gardien de but, soit les deux équipes à la fois.

Il convient de souligner que le nombre de partition du terrain correspond au nombre de joueurs considérés dans l'analyse, chaque partition correspondant à la zone de domination d'un joueur donné et correspond à l'ensemble des points plus proches d'un joueur que des autres joueurs.

L'inconvénient majeur de cette technique est que l'analyse tactique est généralement restreinte à une occupation territoriale de chaque équipe, de manière binaire, c'est-à-dire chaque zone est dominée soit par une équipe, soit par l'équipe adverse, ne répondant pas au besoin d'une mesure de la qualité locale de la domination d'une des deux équipes.

KR 2018 0063777 concerne un procédé d'analyse de matchs de football permettant d'améliorer la tactique d'une équipe en temps réel, mais il ne mentionne aucune détermination d'un vecteur vitesse instantanées ou la détermination d'un temps d'avance d'un joueur par rapport à un autre.

Aucun des systèmes actuels ne permet de répondre simultanément à tous les besoins requis, à savoir de proposer une technique qui permette d'effectuer une analyse plus fine d'une phase de jeu, notamment en prévoyant un mouvement avantageux pouvant par exemple aboutir à un marquage au score par l'une des deux équipes, et de construire des indicateurs de performance reflétant cette analyse fine.

**EXPOSÉ DE L'INVENTION**

La présente invention vise à remédier à tout ou partie des inconvénients de l'état de la technique cités ci-dessus.

À cet effet, l'invention vise, selon un premier aspect, un procédé d'analyse d'une occupation dynamique d'un terrain par une pluralité d'éléments mobiles, un élément mobile étant un joueur d'une équipe parmi deux équipes adversaires d'un jeu ou un accessoire de jeu, tel qu'un ballon, une balle, un palet ou un volant, chaque équipe comprenant au moins un joueur, l'accessoire de jeu étant mobile.

Le jeu peut être par exemple un sport collectif tel que le football, le handball, le water-polo, le basketball, le hockey, le baseball, le cricket, le volley-ball, le football américain ou le rugby. Chaque équipe comprenant alors une pluralité de joueurs.

Le jeu peut également être un sport de raquettes tel que le tennis, le badminton ou le squash. Auquel cas, chaque équipe comprend généralement un ou deux joueurs.

De manière générale, le jeu est codifié par des règles établissant notamment les possibilités et les contraintes à respecter pour chaque joueur, sur le terrain qui est généralement délimité par un marquage.

Il convient de souligner que le terrain est usuellement délimité et comprend un marquage. En outre, ce marquage partage généralement le terrain en deux moitiés de terrain, chaque équipe ayant une moitié du terrain attitré.

Selon les règles de chaque sport, soit chaque équipe est contrainte dans sa moitié de terrain, ce qui est le cas notamment lorsqu'un filet est présent entre les deux moitiés de terrain, soit chaque équipe à la possibilité d'aller dans la moitié de terrain de l'adversaire.

Par soucis de clarté, on entendra par « sport de filet » un sport où chaque équipe est contrainte dans une partie d'un terrain. Un tel sport est par exemple le volley-ball, le tennis ou le badminton.

On entendra par « sport à terrain partagé », un sport où les deux équipes partagent le même terrain. Le terrain peut être divisé en deux moitiés de terrain distinctes comme au football, au rugby, au handball ou au basketball, chaque moitié étant allouée à une équipe. Il convient de souligner que chaque équipe est généralement libre d'occuper la moitié de terrain allouée à l'équipe adverse, dans la limite des règles du sport.

Toutefois, le terrain peut également ne pas être divisé en deux parties distinctes comme au squash.

L'accessoire de jeu utilisé au cours d'une partie est selon les cas soit un ballon, rond ou ovale, une balle, un palet ou un volant. Il convient de souligner que généralement seul un accessoire de jeu est présent sur le terrain afin d'être échangé ou disputé par les deux équipes.

L'accessoire de jeu est généralement disputé dans le cadre d'un sport collectif sur un terrain ayant généralement deux zones de but situées chacune sur un bord opposé du terrain. Chaque équipe défend une des zones de but et va chercher à faire parvenir l'accessoire de jeu dans la zone de but défendue par l'équipe adverse afin de marquer au score.

Dans le cadre d'un sport de raquettes ou d'un sport à filet, l'accessoire de jeu est généralement échangé par les deux équipes jusqu'à que l'échange soit interrompu par une faute commise par l'une ou l'autre des équipes, le point étant alors accordé à l'équipe n'ayant pas commise de faute.

Il convient de souligner que l'on entend ici par accessoire de jeu, tout élément susceptible d'être échangé ou disputé par les deux équipes. L'accessoire de jeu est généralement mobile et distinct d'un accessoire de la panoplie d'un joueur d'une équipe, telle qu'une crosse de hockey ou une batte de baseball.

Par ailleurs, dans la suite de la description, lorsqu'on parlera d'un joueur d'une équipe, on entendra par « joueur adverse », un joueur de l'autre équipe que l'on nommera également par « équipe adverse ».

Le procédé d'analyse d'une occupation dynamique du terrain comprend des étapes de :

- acquisition d'une position instantanée pour tout ou partie des éléments mobiles sur le terrain à au moins deux instants distincts, les éléments mobiles dont la position instantanée est connue étant appelés éléments mobiles considérés ;
- pour chaque élément mobile, détermination d'un vecteur vitesse instantanée ;
- pour chaque élément mobile localisé et pour tout ou partie des points du terrain, détermination d'une durée de déplacement entre la position instantanée dudit élément mobile et ledit point du terrain en fonction dudit vecteur vitesse instantanée, et d'un modèle de déplacement associé audit élément mobile ;
- pour chaque élément mobile localisé et pour tout ou partie des points du terrain, détermination du temps d'avance dudit élément mobile en ledit point du terrain, ledit temps d'avance étant calculé par une comparaison entre la durée de déplacement dudit élément mobile vers ledit point du terrain et la durée de déplacement d'au moins un autre élément mobile vers le même point ;
- pour tout ou partie des joueurs, délimitation d'une zone sur le terrain, dite zone de domination dudit joueur, correspondant à l'ensemble des points du terrain pour lesquels le temps d'avance dudit joueur est supérieur à un seuil prédéterminé.

Ainsi, il est possible d'effectuer une analyse plus fine d'une phase de jeu en délimitant pour un joueur une zone de terrain dans laquelle il domine le jeu par rapport aux joueurs de l'équipe adverse. Le seuil prédéterminé permet notamment de bien délimiter ces zones de terrains avantageuses dans lesquelles les joueurs ont un temps d'avance important. Par exemple, dans le cadre d'un match de football, un temps d'avance peut par exemple être considéré important lorsqu'il est supérieur à 0,5 secondes. Il convient de souligner que la valeur du seuil prédéterminé peut être fonction du sport considéré, voire d'un choix particulier d'un individu, tel qu'un entraineur, souhaitant analyser une phase de jeu.

Il convient de souligner que deux zones de domination de deux joueurs distincts, faisant ou non partis de la même équipe, peuvent ou non se chevaucher, en fonction de la valeur du seuil prédéterminé.

Il convient de souligner que l'analyse est améliorée lorsque le nombre de points du terrain couverts par au moins deux zones de domination diminue.

Avantageusement, aucune zone de domination d'un joueur ne chevauche une autre zone de domination d'un autre joueur.

En d'autres termes, un point du terrain ne peut faire partie que d'une zone de domination d'un joueur.

Par ailleurs, lorsqu'on un point du terrain, aucun joueur n'a un temps d'avance supérieur au seuil prédéterminé, on considère généralement que ce point fait partie d'une zone dite neutre où aucune équipe ne domine.

On entendra par durée de déplacement d'un joueur vers un point du terrain le temps minimum que peut mettre ledit joueur pour atteindre ce point du terrain.

Pour chaque point du terrain et pour chaque élément mobile localisé, la durée de déplacement dudit élément mobile vers ce point peut être calculée à partir de la position et du vecteur vitesse instantanée dudit élément mobile à l'instant considéré, et de paramètres caractéristiques du joueur, en résolvant l'équation d'un modèle de déplacement associé audit élément mobile, selon l'un des procédés bien connus de l'homme de l'art.

Il convient de souligner que la position et la vitesse instantanée des éléments mobiles sont généralement des données réelles acquises au cours d'un match.

Un exemple non limitatif d'équation modélisant le mouvement d'un joueur est fourni par un modèle qui peut s'écrire sous la forme suivante avec v la vitesse du joueur considéré, $v_{max}$ sa vitesse maximale et $\tau$ son temps caractéristique d'accélération :

$$\frac{d\vec{v}}{dt} = \frac{\vec{v}_{max} - \vec{v}}{\tau}$$

Généralement, les paramètres caractéristiques associés à un joueur peuvent être notamment une accélération maximale et une vitesse maximale, ou tout autre paramètre physique associé au joueur permettant d'établir un modèle de course associé au joueur.

La vitesse instantanée d'un joueur est généralement calculée à partir de deux positions instantanées prises à deux instants distincts.

La vitesse instantanée de l'accessoire de jeu mobile peut être calculée à partir à deux positions instantanées prises à deux instants distincts ou déterminé par la vitesse d'une interaction d'un joueur avec l'accessoire de jeu, telle qu'une frappe de ce joueur dans l'accessoire de jeu.

Les paramètres caractéristiques associés à l'accessoire de jeu dépendent généralement du type d'accessoire de jeu. De tels paramètres peuvent comprendre une vitesse maximale, un coefficient de friction, etc.

Pour chaque point du terrain, la durée de déplacement de l'accessoire de jeu vers ce point peut être calculé à partir de la position et du vecteur vitesse instantanée de l'accessoire de jeu à l'instant considéré, en résolvant l'équation modélisant le mouvement de l'accessoire de jeu selon l'un des procédés bien connus de l'homme de l'art. À titre d'exemple non limitatif, le mouvement de l'accessoire de jeu peut être modélisé en considérant que son déplacement s'effectue à vitesse constante. Ainsi, lorsque l'accessoire de jeu est à proximité immédiate d'un joueur, c'est-à-dire que les positions de l'accessoire de jeu et du joueur sont identiques ou à faible distance l'un de l'autre, par exemple inférieur à 10 ou à 20 cm dans le cadre d'un match de football, la durée de déplacement $t_d$ de l'accessoire de jeu vers un point du terrain est obtenu par la formule :

$$t_d = |x_{point} - x_{accessoire}|/v_{accessoire}$$

Dans cette formule, /./ représente la norme euclidienne, $x_{accessoire}$ la position de l'accessoire de jeu, $x_{point}$ la position du point considéré et $v_{accessoire}$ la vitesse maximale pouvant être communiquée à l'accessoire de jeu par le joueur.

Il convient de souligner que pour des raisons de praticité, le terrain est généralement représenté par une matrice de portions généralement carrées ou rectangulaires, équivalents à des pixels d'une image. Chaque portion peut être représentée par un point, dit point du terrain, correspondant par exemple au centre de ladite portion.

Par ailleurs, lorsqu'un élément mobile n'est plus présent sur le terrain, sa durée de déplacement vers un point quelconque du terrain est par exemple déterminée comme égale à l'infini afin que ledit élément mobile ne soit pas considéré dans l'analyse.

Une accélération instantanée peut également être calculée à partir de deux vecteurs vitesses instantanées.

Dans des modes de réalisation particuliers de l'invention, la délimitation de la zone de domination d'un joueur est effectuée sur une image représentative de tout ou partie du terrain.

L'image représentative de tout ou partie du terrain correspond généralement à une vue perpendiculaire au terrain. Le terrain étant généralement horizontal, la vue perpendiculaire correspond à une vue de dessus du terrain.

Toutefois, l'image représentative de tout ou partie du terrain peut être oblique, le terrain étant alors en perspective.

Il convient de souligner que l'image représentative de tout ou partie du terrain peut être schématique ou correspondre à une photo ou à un extrait vidéo. Dans le cas d'une photo ou d'un extrait vidéo, une détermination de la position relative et de l'orientation du terrain par rapport à une caméra ayant acquise l'image ou la vidéo est généralement effectuée.

Dans des modes de réalisation particuliers de l'invention, le procédé comprend également une étape de délimitation du terrain en trois espaces : un espace de domination pour chaque équipe et un espace neutre, l'espace de domination d'une équipe comprenant l'ensemble des zones de domination des joueurs de l'équipe, l'espace neutre correspondant à l'ensemble des points de tout ou partie du terrain non couverts par un espace de domination d'une équipe.

Ainsi, il est possible d'établir une analyse très fine des phases de jeu, ce qui permet notamment de prédire plus efficacement une phase de jeu favorable à l'un des deux adversaires, pouvant aboutir à un marquage au score, comme une action de but favorable dans le cas d'un match de football.

En outre, l'analyse permet notamment de délimiter des espaces neutres où aucune équipe ne domine. Ces espaces, non accessibles par les méthodes de l'art antérieur, permettent d'affiner l'analyse du jeu en mettant en avant par exemple des espaces présentant un risque de perte important de l'accessoire de jeu car dans ces espaces neutres deux joueurs adverses sont généralement trop proches pour que l'un des deux puisse conserver l'accessoire de jeu ou le passer rapidement à un autre joueur de son équipe, sans risque que le joueur adverse ne puisse intercepter l'accessoire de jeu et le récupérer pour son équipe. Ceci est notamment le cas des sports collectifs à terrain partagé. Il convient de souligner que dans l'art antérieur ces espaces seraient considérés comme dominés par l'une des deux équipes, sans tenir compte de la position des joueurs de l'autre équipe.

Par ailleurs, il est également possible de quantifier la domination de chaque joueur de chaque équipe. Pour les sports collectifs à terrain partagé, cette domination est notamment caractérisée en déterminant notamment leur temps d'avance par rapport à un adversaire. Plus le temps d'avance est important, plus le joueur est considéré comme dominant l'espace par rapport à son adversaire direct car il peut y recevoir l'accessoire de jeu et/ou effectuer une action avec l'accessoire de jeu avec un risque faible de perte de l'accessoire de jeu ou de récupération de ce dernier par un joueur de l'équipe adverse. Pour les sports à filets, la domination d'un joueur est quantifiée en chaque point en déterminant son temps d'avance par rapport à l'accessoire de jeu. Plus le temps d'avance est important, plus le joueur est considéré comme dominant l'espace car il peut y préparer son coup de renvoi avant d'y recevoir l'accessoire de jeu, avec par conséquent un risque plus faible de réaliser une faute.

En résumé, une cartographie permettant une caractérisation plus fine de l'occupation dynamique du terrain par chaque adversaire, peut être obtenue. Cette cartographie permet notamment d'améliorer l'analyse tactique de la phase de jeu, en visualisant mieux les zones de domination de chaque équipe.

Dans des modes de réalisation particuliers de l'invention, la délimitation de chaque espace dans la cartographie est fonction d'une comparaison entre les durées de déplacement d'au moins un joueur de chaque équipe.

Avantageusement, l'espace neutre correspond à l'ensemble des points du terrain pour lesquels la différence entre la durée minimale de déplacement de chaque équipe est inférieure en valeur absolue à un seuil prédéterminé, la durée minimale de déplacement de chaque équipe vers un point du terrain correspondant à la valeur minimale des durées de déplacement des joueurs de ladite équipe vers ce point.

En d'autres termes, la différence entre la durée minimale de déplacement de chaque équipe correspond au temps d'avance d'un joueur d'une équipe par rapport à son adversaire direct, c'est-à-dire à son adversaire le plus proche. Ce dernier peut intervenir et récupérer l'accessoire de jeu si celui-ci est envoyé dans cet espace neutre.

Dans des modes de réalisation particuliers de l'invention, l'espace de domination d'un joueur d'une équipe correspond à l'ensemble des points du terrain pour lesquels ledit joueur a un temps d'avance par rapport aux joueurs de l'équipe adverse supérieur à un seuil prédéterminé, le temps d'avance étant égal à la différence entre la durée de déplacement de deux joueurs vers le même point.

Il convient de souligner que les valeurs absolues des seuils prédéterminés pour délimiter les espaces neutres et les espaces de domination sont généralement égales. Un exemple non limitatif de valeur de seuil est 0,1 s.

L'espace de domination d'un joueur d'une équipe correspond alors de même à l'ensemble des points du terrain pour lesquels ledit joueur a un temps d'avance par rapport aux joueurs de l'équipe adverse supérieur à un seuil prédéterminé, le temps d'avance étant égal à la différence entre la durée de déplacement de deux joueurs vers le même point.

Avantageusement, les points du terrain pour lesquels un autre joueur de la même équipe a une durée de déplacement plus faible que ledit joueur vers lesdits points du terrain sont exclus de l'espace de domination d'un joueur d'une équipe.

Il convient de souligner que ces modes de réalisation particuliers de l'invention sont généralement adaptés aux sports à terrain partagé, dans le cas où la position de l'accessoire de jeu n'est ni détectée ni suivie.

Dans des modes de réalisation particuliers de l'invention, l'espace neutre comprend les points de tout ou partie du terrain où la durée de déplacement de l'accessoire de jeu est supérieure à la durée de déplacement d'au moins un joueur de chaque équipe.

Dans des modes de réalisation particuliers de l'invention, une zone de domination d'un joueur d'une équipe correspond à l'ensemble des points du terrain pour lesquels à la fois :

- la durée de déplacement dudit joueur vers un point dudit espace de domination est égale à la plus petite de toutes les durées de déplacement de tous les joueurs vers ce point, et
- la durée de déplacement de l'accessoire de jeu vers le même point dudit espace de domination est inférieure à la plus petite des durées de déplacement des joueurs de l'autre équipe.

Dans des modes de réalisation particuliers de l'invention, les éléments mobiles dont les positions instantanées sont acquises sont uniquement des joueurs, le procédé comprenant également une étape de détermination de la position instantanée de l'accessoire de jeu comme étant égale à la position d'un joueur.

Ainsi, il est possible d'estimer la position instantanée d'un accessoire de jeu sans le détecter à l'image. Il convient de souligner que l'accessoire de jeu peut être caché, voire porté, par un joueur, ce qui est notamment le cas au cours d'un match de rugby.

Lorsque la position instantanée d'un accessoire de jeu est déterminée comme étant égale à la position instantanée d'un joueur, la vitesse instantanée de l'accessoire de jeu est généralement différente de la vitesse de ce joueur, mais correspond plutôt à une vitesse induite par ce joueur dans l'accessoire de jeu. Cette vitesse induite peut être par exemple une vitesse de frappe dans l'accessoire de jeu, une vitesse de passe, etc.

Dans des modes de réalisation particuliers de l'invention, le terrain comprend deux parties de terrain, les déplacements de chaque équipe étant contraints à l'intérieur d'une partie distincte du terrain, et le temps d'avance d'un joueur est calculé par rapport à l'accessoire de jeu, le temps d'avance étant égal à la différence entre la durée de déplacement de l'accessoire de jeu vers ledit point et la durée de déplacement dudit joueur vers ce point.

Dans des modes de réalisation particuliers de l'invention, le temps d'avance d'une équipe en un point de la partie de terrain occupée par ladite équipe correspond au maximum des temps d'avance des joueurs de ladite équipe.

Dans des modes de réalisation particuliers de l'invention, l'espace de domination d'une équipe dans la partie du terrain occupée par l'équipe adverse correspond à l'ensemble des points de ladite partie du terrain pour lesquels le temps d'avance de l'accessoire de jeu est supérieur à un seuil prédéterminé, le temps d'avance de l'accessoire de jeu étant égal à la différence entre la plus petite des durées de déplacement des joueurs de l'équipe adverse et la durée de déplacement de l'accessoire de jeu vers ledit point.

Dans des modes de réalisation particuliers de l'invention, la délimitation de chaque espace dans la cartographie est fonction d'une comparaison entre la durée de déplacement d'au moins un joueur et une durée de déplacement de l'accessoire de jeu, la durée de déplacement de l'accessoire de jeu étant déterminée à partir d'une position instantanée de l'accessoire de jeu préalablement acquise et d'un modèle de mouvement associé audit accessoire de jeu.

Ainsi, il est possible de raffiner la délimitation des espaces de domination et des espaces neutres en tenant compte de la position instantanée de l'accessoire de jeu.

Dans d'autres modes de réalisation particuliers de l'invention, la délimitation de chaque espace dans la cartographie est fonction d'une comparaison entre la durée de déplacement d'au moins un joueur et une durée de déplacement de l'accessoire de jeu, la durée de déplacement de l'accessoire de jeu étant déterminée en supposant sa position égale à la position instantanée d'un joueur distinct dudit au moins un joueur.

Ainsi, il est possible d'effectuer l'analyse sans connaître la position instantanée de l'accessoire de jeu, mais en considérant une position hypothétique de l'accessoire de jeu comme égale à la position d'un joueur. Ceci est notamment utile lorsque l'accessoire de jeu ne peut être détecté ni suivi dans un flux vidéo.

Dans des modes de réalisation particuliers de l'invention, l'espace neutre correspond aux points où la durée de déplacement de l'accessoire de jeu est supérieure à la durée de déplacement d'au moins un joueur de chaque équipe.

Une zone de domination d'un joueur d'une équipe correspond alors à l'ensemble des points du terrain pour lesquels à la fois :

- la durée de déplacement dudit joueur vers un point dudit espace de domination est égale à la plus petite de toutes les durées de déplacement de tous les joueurs vers ce point, et
- la durée de déplacement de l'accessoire de jeu vers le même point dudit espace de domination est inférieure à la plus petite des durées de déplacement des joueurs de l'autre équipe.

Ces modes de réalisation particuliers de l'invention sont en général adaptés aux sports à terrain partagé, lorsque la position de la balle est détectée et suivie.

Dans d'autres modes de réalisation particuliers de l'invention, une zone de domination d'un joueur d'une équipe dans sa partie du terrain correspond à l'ensemble des points de sa partie du terrain pour lesquels à la fois :

- la durée de déplacement dudit joueur vers un point dudit espace de domination est égale à la plus petite de toutes les durées de déplacement de tous les joueurs dans ladite partie du terrain vers ce point, et
- la durée de déplacement de l'accessoire de jeu vers le même point dudit espace de domination est supérieure à celle dudit joueur.

Une zone de domination d'un joueur d'une équipe dans la partie adverse du terrain correspond alors à l'ensemble des points de la partie adverse du terrain pour lesquels la durée de déplacement de l'accessoire de jeu depuis la position dudit joueur vers le point dudit espace de domination est inférieure à la plus petite de toutes les durées de déplacements de tous les joueurs de la partie adverse du terrain. La zone de domination d'une équipe dans la partie adverse du terrain correspond alors à l'ensemble des zones de domination des joueurs d'une équipe dans la partie adverse du terrain.

La zone neutre d'une partie terrain occupée par une équipe correspond alors à l'ensemble des points de ladite partie du terrain pour lesquels la durée de déplacement de l'accessoire de jeu vers ce point est similaire à la plus petite des durées de déplacements des joueurs de l'équipe occupant cette partie du terrain vers ce point, c'est-à-dire que la valeur absolue de la différence entre les deux durées minimales est inférieure à un seuil prédéterminé.

En d'autres termes, la zone neutre correspond aux points où le temps minimal d'envoi de l'accessoire de jeu par l'équipe adverse vers ce point est proche du temps de déplacement minimal des joueurs de l'équipe occupant cette partie du terrain. Un seuil prédéterminé d'écart maximal entre les deux temps permet de déterminer si ces temps sont proches ou non. Un exemple non limitatif de seuil est 0,1 s

Dans des modes de réalisation particuliers de l'invention, la durée de déplacement de l'accessoire de jeu vers un point d'une partie du terrain considérée pour définir la zone de domination d'un joueur de l'équipe occupant ladite partie du terrain, pour définir la zone de domination de l'équipe adverse et pour définir la zone neutre est la plus petite des durées de déplacement de l'accessoire de jeu depuis l'une des positions des joueurs adverses vers ce point.

Ainsi, il est possible d'effectuer l'analyse sans connaître la position instantanée de l'accessoire de jeu, mais en considérant une position hypothétique de l'accessoire de jeu comme égale à la position d'un joueur adverse, ce joueur adverse étant choisi comme celui associé à la durée de déplacement de l'accessoire de jeu la plus faible. Ceci est notamment utile lorsque l'accessoire de jeu ne peut être détecté ni suivi dans un flux vidéo.

Il convient de souligner que ces modes de réalisation particuliers de l'invention sont en général adaptés aux sports à filets.

Avantageusement, l'espace de domination d'une équipe correspond à l'ensemble des zones de domination des joueurs de ladite équipe

Dans des modes de réalisation particuliers de l'invention, le procédé comprend également une étape de détermination d'une valeur représentative de la domination d'un joueur en un point du terrain, ladite valeur étant égale à la différence entre la plus petite des durées de déplacement des joueurs de l'autre équipe vers ledit point et la durée de déplacement dudit joueur vers ledit point. Ces modes de mise en oeuvre sont généralement adaptés aux sports à terrains partagés, lorsque l'accessoire de jeu ne peut être détecté ni suivi.

Dans des modes de mise en oeuvre particuliers de l'invention, le procédé comprend également une étape de détermination d'une valeur représentative de la domination d'un joueur en un point du terrain, ladite valeur étant égale au minimum entre :

- la différence entre la plus petite des durées de déplacement des joueurs de l'autre équipe vers ledit point et la durée de déplacement dudit joueur vers ledit point d'une part,
- la différence entre la plus petite des durées de déplacement des joueurs de l'autre équipe vers ledit point et la durée de déplacement de l'accessoire de jeu vers ledit point d'autre part.

Avantageusement dans ces modes de réalisation particuliers, la valeur représentative de la domination est fixée à zéro pour l'espace neutre.

Ces modes de réalisation particuliers de l'invention sont généralement adaptés aux sports à terrains partagés, lorsque l'accessoire de jeu peut être détecté et suivi.

Dans d'autres modes de réalisation particuliers de l'invention, le procédé comprend également une étape de détermination d'une valeur représentative de la domination d'un joueur en un point de sa partie du terrain, ladite valeur étant égale à la différence entre la durée de déplacement de l'accessoire de jeu vers ledit point et la durée de déplacement dudit joueur vers ledit point.

Avantageusement dans ces modes de réalisation particuliers, la valeur représentative de la domination d'une équipe en un point de sa partie du terrain correspond au maximum des valeurs représentatives de la domination des joueurs de cette équipe.

Dans ces modes de réalisation particuliers, le procédé comprend également une étape de détermination d'une valeur représentative de la domination d'une équipe en un point de la partie du terrain occupée par l'équipe adverse, ladite valeur étant égale à la différence entre la plus petite des durées de déplacement des joueurs adverses vers ledit point et la durée de déplacement de l'accessoire de jeu vers ledit point. Il convient de souligner ici que les valeurs représentatives de la domination d'une équipe et de l'autre dans la même partie du terrain sont opposées l'une de l'autre. Ces modes de mise en oeuvre sont généralement adaptés aux sports à filets.

Ces modes de réalisation particuliers de l'invention sont généralement adaptés aux sports à filets.

Dans des modes de réalisation particuliers de l'invention, la durée de déplacement de l'accessoire de jeu vers un point d'une partie du terrain considéré pour définir la valeur représentative de domination d'un joueur de l'équipe ou d'une équipe occupant ladite partie du terrain, ou pour définir la valeur représentative de l'équipe adverse, est la plus petite des durées de déplacement de l'accessoire de jeu depuis l'une des positions des joueurs adverses vers ce point. Ainsi, il est possible d'effectuer l'analyse sans connaître la position instantanée de l'accessoire de jeu, mais en considérant une position hypothétique de l'accessoire de jeu comme égale à la position d'un joueur adverse, ce joueur adverse étant choisi comme celui associé à la durée de déplacement de l'accessoire de jeu la plus faible. Ceci est notamment utile lorsque l'accessoire de jeu ne peut être détecté ni suivi dans un flux vidéo.

Dans des modes de réalisation particuliers de l'invention, le procédé comprend également une étape de détermination d'une valeur représentative de la domination d'un joueur en un point de la partie adverse du terrain, ladite valeur étant égale à la différence entre la plus petite des durées de déplacement des joueurs de l'équipe et la durée de déplacement de l'accessoire de jeu depuis la position dudit joueur vers ledit point.

Ces modes de réalisation particuliers de l'invention sont généralement adaptés aux sports à filets.

Dans des modes de réalisation particuliers de l'invention, le procédé comprend une étape de détermination d'une intensité de couleur fonction de la domination du joueur.

Ainsi, les espaces de domination de chaque équipe peuvent être sous-divisées en zones de domination importante et en zones de domination faible. Généralement les zones de domination importante ont une intensité plus foncée que les zones de domination faible afin de créer un dégradé de couleur entre une zone neutre et une zone de domination importante.

Il convient de souligner que la qualité de la domination peut être calculée en tout point du terrain et que les valeurs de domination peuvent être représentées sous la forme d'une surface continue et différentiable.

Généralement chaque adversaire est représenté par une couleur distincte (par exemple : rouge et bleu). Les zones neutres sont également représentées avec une autre couleur (par exemple : blanc).

Dans des modes de réalisation particuliers de l'invention, la génération de la cartographie est effectuée en temps réel.

Il convient de souligner que la génération de la cartographie est généralement effectuée en temps réel par rapport à la diffusion du flux vidéo qui peut être diffusé en direct ou en différé.

On entend par temps réel un traitement effectué quasi-instantanément, généralement inférieur à quelques dixièmes de secondes afin que la cartographie puisse être synchronisée avec l'image du flux vidéo affiché quasiment en direct à l'écran.

Dans des modes de réalisation particuliers de l'invention, le procédé comprend également une étape d'acquisition d'un flux vidéo par au moins une caméra couvrant tout ou partie du terrain, l'étape d'acquisition de la position instantanée de l'accessoire de jeu comprend une sous-étape d'analyse du flux vidéo pour déterminer ladite position instantanée.

Préférentiellement, chaque caméra est fixe et couvre tout ou partie du terrain.

Avantageusement, l'ensemble des caméras couvre l'intégralité du terrain.

Dans des modes de réalisation particuliers de l'invention, l'étape d'acquisition de la position instantanée de chaque joueur comprend une sous-étape d'analyse du flux vidéo pour déterminer ladite position instantanée.

Dans des modes de mise en oeuvre particuliers de l'invention, la cartographie est synchronisée et superposée sur le flux vidéo.

Ainsi, il est possible de voir en temps réel une image augmentée du terrain, en d'autres termes une image du terrain sur laquelle est incrustée les espaces de domination de chaque équipe, voire les espaces neutres. Des informations additionnelles telles que les valeurs représentatives de la qualité de domination au sein des zones de domination peuvent également être incrustés à l'image.

Dans des modes de réalisation particuliers de l'invention, le procédé comprend une étape de suivi de tout ou partie des joueurs et/ou de l'accessoire de jeu dans le flux vidéo.

Ainsi, il est possible de déterminer la position instantanée de tout ou partie des joueurs et/ou de l'accessoire de jeu dans le flux vidéo.

Dans des modes de réalisation particuliers de l'invention, l'étape de détermination de la position de chaque joueur sur le terrain comprend une sous-étape d'analyse d'un signal émis par une balise solidarisée audit joueur et reçu par au moins une borne située à proximité du terrain.

Préférentiellement, les signaux émis par les balises sont reçus par au moins trois bornes situées à proximité du terrain.

Dans des modes de réalisation particuliers de l'invention, le procédé comprend également une étape de détermination d'au moins un indicateur quantitatif d'une caractéristique qualitative d'un joueur choisie parmi :

- une valeur représentative de la domination du joueur dans au moins une partie du terrain, calculée à partir des caractéristiques des zones de domination enregistrées au cours d'un match ;
- une qualité de passe de l'accessoire de jeu ;
- une qualité de domination individuelle d'une portion du terrain
- une valeur représentative de la pression subie par ledit joueur ;
- une qualité de frappe dans l'accessoire de jeu ;
- le taux de fautes directes.

Dans des modes de réalisation particuliers de l'invention, le procédé comprend également une étape de détermination d'au moins un indicateur quantitatif d'une caractéristique qualitative d'une équipe, construite par l'agrégation des indicateurs individuels de ses joueurs.

Dans des modes de réalisation particuliers de l'invention, ces indicateurs quantitatifs de performance peuvent être calculés et affichés et/ou enregistrés sans qu'il y ait nécessairement affichage des cartographies de domination dynamiques associées.

Dans des modes de réalisation particuliers de l'invention, le procédé comprend également des étapes de :

- affichage en temps réel du flux vidéo sur lequel est superposé au moins un indicateur de suivi d'un joueur ; et
- repositionnement de l'indicateur de suivi par un opérateur.

Ainsi, une erreur de suivi peut être corrigée rapidement. Il convient en effet de souligner que le suivi d'un joueur étant généralement basé sur le suivi du contour de l'image du joueur dans le flux vidéo, il arrive que le suivi du contour passe sur un autre joueur lorsque les images des deux joueurs se superposent dans le flux vidéo.

Selon un deuxième aspect, l'invention vise un système d'analyse d'une occupation dynamique d'un terrain par deux équipes adversaires d'un jeu, mettant en oeuvre le procédé de génération d'une cartographie selon l'un quelconque des modes de mise en oeuvre précédents.

Le système d'analyse d'une occupation dynamique du terrain comprend notamment :

- des moyens d'acquisition de la position instantanée de chaque joueur sur le terrain ;
- des moyens d'acquisition de la position instantanée de l'accessoire de jeu sur le terrain ;
- des moyens informatiques de traitement de données comprenant un processeur et une mémoire informatique ;
- des moyens d'affichage graphique de la cartographie générée et des indicateurs associés.

Dans des modes de réalisation particuliers de l'invention, les moyens d'acquisition de la position instantanée comprennent au moins une caméra acquérant un flux vidéo couvrant tout ou partie du terrain.

Dans des modes de réalisation particuliers de l'invention, les moyens d'acquisition de la position instantanée de chaque joueur et/ou de l'accessoire de jeu comprennent au moins une balise et au moins une borne de réception d'un signal émis par ladite balise.

Avantageusement, au moins une balise peut notamment être solidarisée à un joueur et/ou à l'accessoire de jeu. Une balise peut également être utilisée pour déterminer les limites du terrain.

Préférentiellement, les moyens d'acquisition comprennent au moins trois bornes de réception.

Dans des modes de réalisation particuliers de l'invention, les moyens d'acquisition de la position instantanée de l'accessoire de jeu comprennent au moins une caméra acquérant tout ou partie du terrain.

Avantageusement, l'ensemble des caméras permettant d'acquérir la position instantanée de chaque joueur est identique à l'ensemble des caméras permettant d'acquérir la position instantanée de l'accessoire de jeu.

En d'autres termes, le flux vidéo acquis par la ou les caméra(s) est utilisé à la fois pour acquérir les positions instantanées de chaque joueur et de l'accessoire de jeu sur le terrain.

Dans des modes de réalisation particuliers de l'invention, une alerte est affichée en temps réel si une action particulière est détectée, comme par exemple lorsqu'un espace est dominé par l'adversaire dans la surface de réparation au football, permettant à l'équipe de mieux suivre sa gestion de l'espace.

Elle permet par exemple d'informer l'équipe d'une possibilité d'action favorable avant qu'elle ne se produise.

Dans des modes de réalisation particuliers de l'invention, le système comprend également des moyens d'enregistrement des données acquises et de modification par un utilisateur de tout ou partie des données enregistrées de positions instantanées et/ou vitesses des éléments mobiles.

Ainsi, il est possible de modifier des paramètres enregistrés en vue d'améliorer la tactique de jeu. Par exemple, la vitesse d'au moins un joueur peut être modifiée, en norme et/ou en orientation, afin de voir l'impact sur la phase de jeu en cours. Cela permet ainsi de répondre à une question du type : « quelle aurait été la domination dynamique de l'espace si ... ? » (par exemple, quelle aurait été la domination dynamique du terrain si l'un des joueurs avait eu une à cet instant une vitesse instantanée dix fois plus faible)

**BRÈVE DESCRIPTION DES FIGURES**

D'autres avantages, buts et caractéristiques particulières de la présente invention ressortiront de la description non limitative qui suit d'au moins un mode de réalisation particulier des dispositifs et procédés objets de la présente invention, en regard des dessins annexés, dans lesquels :

- la figure 1 est un schéma d'ensemble représentant un système d'analyse de l'occupation dynamique d'un terrain par deux équipes adversaires d'un jeu de football ;
- la figure 2 est un schéma synoptique d'un procédé d'analyse mis en oeuvre par le système d'analyse de la figure 1 ;
- la figure 3 est une comparaison de deux cartographies obtenues soit par le procédé d'analyse de la figure 2 (figure 3A), soit par les méthodes de l'art antérieur (figure 3B) ;
- la figure 4 est une cartographie des durées de déplacements individuelle de l'un des joueurs sur le terrain, obtenue au cours du procédé de la figure 2, les durées de déplacements étant utilisées pour obtenir la cartographique de la figure 3A ;
- la figure 5 est une comparaison de deux cartographies obtenues par le procédé d'analyse de la figure 2 afin d'analyser l'évolution de la tactique en fonction d'un scénario donné, la cartographie de la figure 5A étant calculée à partir des positions et vitesses instantanées acquises, la cartographie de la figure 5B étant calculée à partir des positions et vitesses instantanées acquises en tenant compte d'une modification d'une vitesse instantanée d'un joueur ;

- la figure 6 est un exemple de cartographie obtenue par un autre exemple de mode de mise en oeuvre du procédé d'analyse de l'occupation dynamique selon l'invention ;
- la figure 7 est un agrandissement d'une partie de la cartographie de la figure 6 ;
- la figure 8 est un schéma synoptique du procédé d'analyse ayant pour résultat la cartographie de la figure 6 ;
- la figure 9 est un autre exemple de cartographie obtenue par un procédé d'analyse de l'occupation dynamique selon l'invention.

## DESCRIPTION DÉTAILLÉE DE L'INVENTION

La présente description est donnée à titre non limitatif, chaque caractéristique d'un mode de réalisation pouvant être combinée à toute autre caractéristique de tout autre mode de réalisation de manière avantageuse.

On note, dès à présent, que les figures ne sont pas à l'échelle.

Exemple d'un mode de réalisation particulier

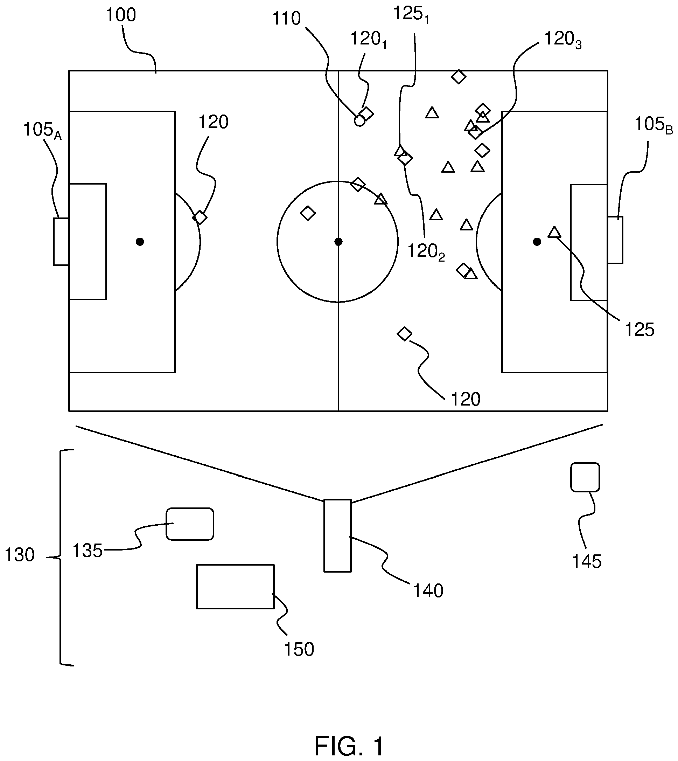

La figure 1 représente un schéma d'un terrain 100 de jeu de football vu du dessus, sur lequel deux équipes adversaires s'affrontent et se disputent un accessoire de jeu mobile qui est dans le présent exemple un ballon 110 de football. Chaque équipe comprenant onze joueurs a pour objectif d'envoyer le ballon 110 dans une des deux zones de but 105 située chacune sur un bord latéral opposé du terrain 100.

Il convient de souligner que le football se joue intégralement à l'intérieur de la ligne extérieure du marquage du terrain 100. Dès lors où le ballon 110 sort du marquage du terrain 100, le jeu est interrompu.

Par soucis de clarté sur la figure 1, les joueurs 120 d'une des deux équipes, appelée équipe A par la suite, sont représentés par des losanges défendent la zone de but $105_A$ située sur la figure 1 sur le bord latéral gauche du terrain 100, tandis que les joueurs 125 de l'autre équipe, appelée équipe B par la suite, correspondant à l'équipe adverse, sont représentés par des triangles.

Les joueurs 120 de l'équipe A ont pour objectif d'envoyer le ballon 110 dans la zone de but $105_B$ située sur le bord latéral droit du terrain 100 représenté en figure 1 et défendue par les joueurs 125 de l'équipe B. Inversement, les joueurs 125 de l'équipe B ont pour objectif d'envoyer le ballon 110 dans la zone de but $105_A$ défendue par l'équipe A. À chaque fois que le ballon 110 entre intégralement dans l'une des zones de but 105, l'équipe n'ayant pas à défendre cette zone de but marque au score.

Afin d'analyser au moins une phase de jeu et en vue d'améliorer la tactique de jeu de chaque équipe, un système 130 d'analyse d'une occupation dynamique du terrain 100 selon l'invention est utilisé.

Il convient de souligner que le terrain 100 est subdivisé pour l'analyse en une pluralité de portions généralement carrés (non représentés sur la figure 1), pouvant également être appelés pixels. Plus la taille des portions du terrain 100 est petite, plus l'analyse de l'occupation du terrain 100 est fine. Chaque portion du terrain 100 est représentée par un point, qui correspond généralement au centre de la portion. Par la suite, on entendra par point le centre d'une portion du terrain 100.

Le système 130 d'analyse comprend notamment des moyens d'acquisition de la position instantanée de chaque joueur 120 et 125 et du ballon 110 sur le terrain 100. Ces moyens d'acquisition comprennent dans le présent exemple non limitatif de l'invention une caméra 140 acquérant un flux vidéo couvrant l'intégralité du terrain 100. Il convient de souligner qu'un flux est une succession d'images fixes, généralement prises avec une cadence comprise entre vingt-cinq images par seconde et cent images par seconde.

Afin de minimiser le traitement des images du flux vidéo, la caméra 140 est avantageusement fixe.

Dans des variantes de ce mode de réalisation particulier de l'invention, les moyens d'acquisition de la position instantanée de chaque joueur 120 et 125 et du ballon 110 sur le terrain 100 comprennent une pluralité de caméras fixes acquérant chacune un flux vidéo couvrant une partie du terrain 100. L'intégralité du terrain 100 est ainsi couverte par une pluralité de caméras. Les positions des joueurs obtenues par chaque caméra sont ensuite rassemblées. L'utilisation d'une pluralité de caméras permet généralement d'obtenir des positions plus précises des joueurs sur le terrain 100, la détermination de la position étant fonction de la résolution de l'image obtenue par la caméra.

Les images acquises par la caméra 140 sont traitées par des moyens informatiques 135 de traitement de données du système 130 d'analyse afin de déterminer la position instantanée de chaque joueur 120 et 125 et/ou du ballon 110 sur le terrain 100.

À cet effet, les moyens de traitement informatique comprennent notamment un processeur et une mémoire informatique.

Afin d'améliorer ou de déterminer la position instantanée de tout ou partie des joueurs 120 et 125 sur le terrain 100, le système 130 d'analyse peut également comprendre une borne 145 de réception d'un signal de présence émis par au moins une balise (non représentée sur la figure 1) solidarisée à tout ou partie des joueurs 120 et 125, et/ou au ballon 110.

Ces balises peuvent notamment être des balises de géolocalisation de type GPS émettant à intervalles réguliers un signal de présence transmettant une identification associée au joueur sur lequel la balise est solidarisée et la position géographique de la balise au moment de l'émission du signal de présence.

Dans des variantes de ce mode de réalisation particulier de l'invention, le système 130 d'analyse comprend trois bornes de réception afin de localiser par triangulation ou par trilatération la position d'une balise émettant un signal de présence.

À cet effet, le signal de présence émis par chaque balise peut avantageusement être modulé selon la technique UWB (acronyme du terme anglais « *Ultra Wide Band* ») afin de réduire les erreurs de mesure du temps de propagation du signal de présence reçus par les bornes de réception.

Il convient de souligner que l'acquisition de la position instantanée des joueurs 120 et 125 sur le terrain 100 peut être effectuée grâce à la présence des balises solidarisées aux joueurs 120 et 125 sans traitement des images acquises par la caméra 140. Toutefois, l'acquisition de la position instantanée par le traitement des images peut être avantageuse lorsqu'au moins un joueur d'un des adversaires présents sur le terrain 100 ne porte pas de balise.

La mémoire informatique stocke notamment un programme d'ordinateur comprenant des instructions mettant en oeuvre un procédé d'analyse, selon l'invention, de l'occupation dynamique du terrain 100 par les deux équipes adversaires afin de générer une cartographie représentative de cette occupation et des indicateurs de performance associés à partir de la position instantanée des joueurs 120 et 125 et du ballon 110 sur le terrain 100.

La cartographie générée et des indicateurs de performance associés sont ensuite affichés sur un écran 150 des moyens d'affichage du système 130 d'analyse.

La cartographie permet de montrer de manière visuelle la qualité de domination en tout point du terrain par l'une ou l'autre des équipes, voire par aucune. Le terrain peut ainsi être subdivisé en au moins trois espaces, dont un espace de domination pour chaque équipe et un espace neutre où aucune équipe n'est dominante.

On entendra par qualité de la domination en un point une valeur représentative de la domination par une équipe en ce point. Cette valeur est calculée par rapport au temps d'avance d'un joueur de l'équipe par rapport au joueur de l'équipe adverse le plus proche.

En fonction du temps d'avance, chaque point du terrain peut être associé à un espace de domination d'une des équipes si le temps d'avance de l'une des équipes est important, c'est-à-dire supérieur à une valeur seuil prédéterminée (par exemple de 0,1 s). Dans le cas où aucun joueur d'une équipe n'a, en ce point, un temps d'avance significativement important par rapport aux autres joueurs, c'est-à-dire que le maximum du temps d'avance est inférieur à la valeur seuil, le point est considéré comme associé à l'espace neutre.

Grâce à cette délimitation astucieuse du terrain, il est ainsi possible d'obtenir une analyse fine de chaque phase de jeu en vue d'améliorer la tactique de jeu d'au moins une équipe.

Les indicateurs de performance peuvent notamment être :

- la qualité individuelle d'un joueur ou collective de domination du terrain entier ou d'une zone spécifique du terrain (par exemple la zone proche d'un but) en un instant, estimée par exemple par la portion de cette zone qui est dominée par ledit joueur ou ladite équipe ainsi que par la valeur caractéristique de la qualité de la domination dans cette zone ;

- la qualité individuelle d'un joueur ou collective de domination d'une zone spécifique du terrain (par exemple la zone proche d'un but) sur une partie du match, estimée par exemple par le nombre de séquences de jeu et le temps total pendant lesquels une portion d'une zone spécifique a été dominée par ledit joueur ou ladite équipe ;

- la qualité de réalisation d'une passe ou de l'ensemble des passes d'un joueur ou d'une équipe, associée à la qualité de la domination au point d'arrivée de la passe ;

- les opportunités de passes possibles à chaque instant pendant tout ou partie du match ;

- une valeur représentative de la pression exercée sur le porteur du ballon.

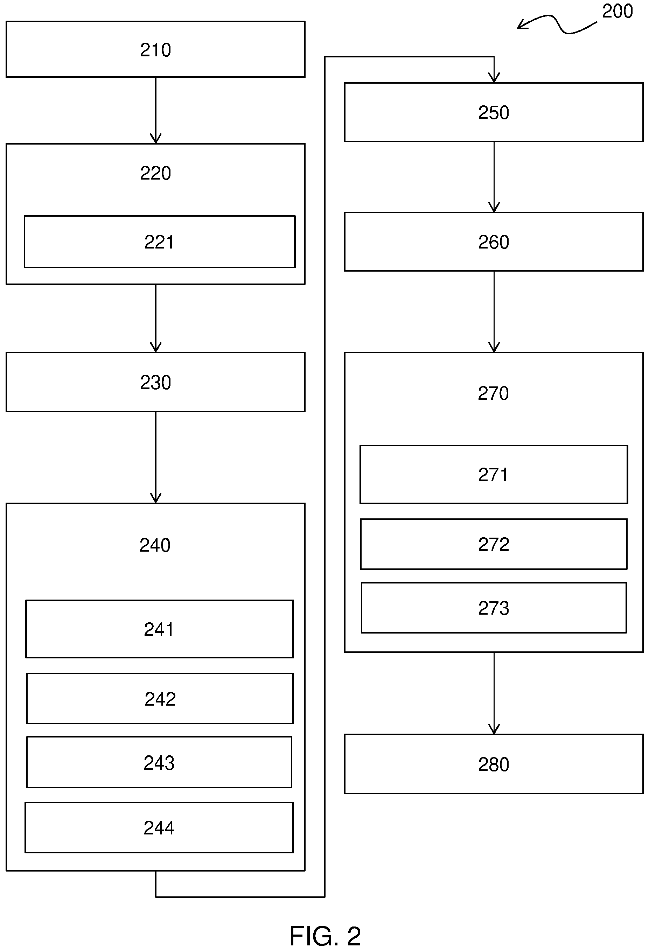

La figure 2 représente sous la forme d'un schéma synoptique le procédé 200 d'analyse de l'occupation dynamique du terrain selon l'invention.

Le procédé 200 comprend dans le présent exemple non limitatif de l'invention une étape 210 d'acquisition d'un flux vidéo par la caméra 140.

Le flux vidéo acquis est ensuite traité au cours de l'étape 220 d'acquisition de la position instantanée du ballon 110 sur le terrain 100.

À cet effet, l'étape 220 comprend une sous-étape 221 d'analyse du flux vidéo pour déterminer la position instantanée du ballon 110. Cette analyse est notamment effectuée par une détection du ballon 110 dans une image du flux vidéo et par un suivi du ballon 110 dans les images successives du flux vidéo. Le suivi est généralement effectué par l'intermédiaire d'un contour venant épouser la forme du ballon 110 ou par toutes autres techniques bien connues de l'homme du métier.

Il convient de souligner que lorsque le ballon 110 est situé en dehors du terrain 100, le jeu étant arrêté, la présente analyse est généralement suspendue le temps que le ballon 110 revienne sur le terrain 100 afin que le jeu puisse reprendre.

À partir de la position instantanée du ballon 110 sur le terrain 100, il est possible de déterminer une durée minimale de déplacement du ballon 110 entre sa position instantanée et un point quelconque du terrain 100. Pour effectuer ce calcul, une vitesse maximale du ballon 110 est généralement considérée. La valeur de la vitesse maximale du ballon 110 est représentative d'une vitesse généralement constatée lors d'un match de football. Il s'agit d'un paramètre pouvant être déterminé en début d'analyse par un opérateur ou adapté au cours de l'analyse en fonction de la vitesse maximale relevée au cours du jeu. La vitesse du ballon 110 peut notamment être déterminée à partir de positions successives du ballon 110 dans les images du flux vidéo.

La détermination d'une durée minimale de déplacement du ballon 110 est effectuée au cours de l'étape 230 du procédé 200 pour tout ou partie des points du terrain.

Par ailleurs, le flux vidéo acquis par la caméra 140 peut également être traité au cours de l'étape 240 d'acquisition de la position instantanée de chaque joueur 120 et 125 sur le terrain 100.

À cet effet, l'étape 240 comprend une sous-étape 241 d'analyse du flux vidéo pour déterminer la position instantanée de chaque joueur 120 et 125. Cette analyse permet de détecter la présence des joueurs 120 et 125 et de les suivre dans les images successives du flux vidéo. Comme pour le suivi du ballon 110, le suivi de chaque joueur est généralement effectué par l'intermédiaire d'un contour venant épouser la forme du joueur ou par toutes autres techniques bien connues de l'homme du métier.

Il convient de souligner que la détection ne reconnaît généralement pas le joueur car effectuée sur une forme générale afin que le suivi puisse être effectué en temps réel.

Afin de pallier des erreurs de suivi, par exemple quand deux contours se superposent, l'étape 240 peut également comprendre une sous-étape 242 permettant à un opérateur d'intervenir et de corriger le suivi des joueurs affiché sur un écran de contrôle. L'écran de contrôle affiche à cet effet le flux vidéo acquis par la caméra 140 sur lequel est superposé un indicateur de suivi par joueur, l'opérateur pouvant repositionner au moins un indicateur si nécessaire.

Il convient de souligner qu'une tierce personne ne faisant partie d'aucune des deux équipes, à savoir un arbitre, est généralement également présente sur le terrain 100. Cette tierce personne n'est avantageusement pas considérée dans la présente analyse d'occupation dynamique du terrain. L'opérateur peut également supprimer le suivi de cette tierce personne dans les images du flux vidéo.

Dans le cas où la tierce personne porte une balise, la position obtenue associée à la balise n'est pas prise en compte dans l'analyse.

L'étape 240 peut également comprendre une sous-étape 243 d'acquisition des signaux émis par les balises solidarisées aux joueurs.

À partir des données issues des images de la caméra et/ou des balises des joueurs, la position instantanée de chaque joueur 120 et 125 sur le terrain est déterminée au cours d'une sous-étape 244. Il convient de souligner que lorsqu'un joueur n'est pas présent sur l'image, ou est situé en dehors du terrain de telle sorte qu'il ne participe plus au jeu, il est indiqué « hors terrain », et n'est pas considéré dans la suite de l'analyse.

Pour chaque joueur présent sur le terrain 100, un vecteur vitesse instantanée est déterminé au cours d'une étape 250 du procédé 200, à partir de deux positions successives dudit joueur. Ce vecteur comprend une direction, correspondant à la direction de déplacement dudit joueur et une norme correspondant à la vitesse instantanée dudit joueur.

En outre, la durée de déplacement entre la position instantanée de chaque joueur et chaque point du terrain est déterminée au cours d'une étape 260 du procédé 200, en fonction du vecteur vitesse instantanée, et de paramètres physiques associés à chaque joueur, comme une accélération et une vitesse maximales. Lorsqu'un joueur est considéré « hors terrain », la durée de déplacement associée vers un point quelconque du terrain est dans le présent exemple non limitatif de l'invention égale à l'infini.

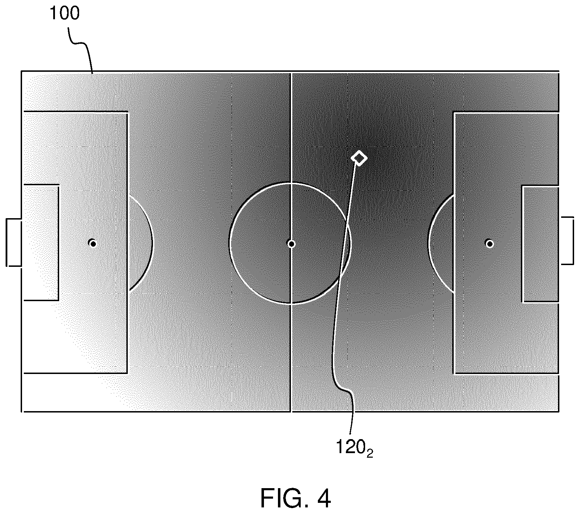

Un exemple de cartographie de la durée de déplacement du joueur $120_2$ sur le terrain 100 est illustré en figure 4.

Pour chaque point du terrain 100, un temps d'avance est calculé pour chaque joueur au cours d'une étape 265 en comparant la durée de déplacement des joueurs vers le même point. Le temps d'avance en un point d'un joueur par rapport à un autre joueur est égal à la différence entre la durée de déplacement des deux joueurs vers le même point.

Le procédé comprend ensuite une étape 270 de délimitation du terrain 100 en trois espaces, dont une zone de domination pour chaque équipe et une zone neutre où aucune équipe n'est dominante.

L'espace de domination de chaque équipe est calculé au cours d'une sous-étape 271 de l'étape 270. La zone de domination d'un joueur d'une équipe correspond à l'ensemble des points du terrain 100 pour lesquels le temps d'avance dudit joueur est supérieur à un seuil prédéterminé.

Avantageusement, la zone de domination d'un joueur d'une équipe correspond à l'ensemble des points du terrain 100 pour lesquels à la fois :

- la durée de déplacement dudit joueur vers un point dudit espace de domination est égale à la plus petite de toutes les durées de déplacement de tous les joueurs vers ce point, et
- la durée de déplacement de l'accessoire de jeu vers le même point dudit espace de domination est inférieure à la plus petite des durées de déplacement des joueurs de l'autre équipe.

En d'autres termes, le temps d'avance d'un joueur en un point est calculé par rapport aux autres joueurs, notamment aux joueurs de l'équipe adverse, en tenant compte également de la durée de déplacement de l'accessoire de jeu en ce point.

Il convient de souligner que l'espace de domination d'une équipe correspond à l'ensemble des zones de domination associées aux joueurs de ladite équipe.

Pour l'affichage de la cartographie, l'espace de domination associé à l'équipe des joueurs 120 est par exemple associé à la couleur bleue. Tandis qu'une couleur distincte, par exemple la couleur rouge, est associée à l'espace de domination de l'équipe des joueurs 125.

Avantageusement, l'intensité de la couleur des points de chaque espace de domination est calculée en fonction de la domination du joueur au cours d'une sous-étape 272 de l'étape 270. La couleur est plus intense lorsque le joueur d'une équipe a un temps d'avance important par rapport aux joueurs de l'autre équipe, en d'autres termes lorsque la durée de déplacement du joueur d'une équipe est notablement inférieure à celle des joueurs de l'autre équipe.

Par exemple, une échelle d'intensité de couleur, comprenant cent-une nuances de couleur bleues, est associée à une plage de temps d'avance comprise entre 0 et 3 secondes (s). Cette plage est ainsi découpée en cent-une catégories de temps d'avance (entre 0 s et 0,03 s, entre 0,03 s et 0,06 s, etc.). L'échelle va du plus clair (quasi-blanc) pour la catégorie de temps d'avance la plus faible, au plus foncé pour la catégorie de temps d'avance la plus élevée. La valeur de couleur du pixel en un point de la représentation graphique de la cartographie est obtenue, si le temps d'avance est inférieur ou égal à 3 s, à la nuance d'intensité associée dans l'échelle. Si le temps d'avance est supérieur à 3 s, on attribue au pixel l'intensité maximale de l'échelle.

L'étape 270 comprend également une sous-étape 273 de détermination d'un espace neutre qui correspondant aux points où la durée minimale de déplacement de l'accessoire de jeu est supérieure à la durée de déplacement d'au moins un joueur de chaque équipe.

Il convient de souligner qu'un joueur d'une équipe pourrait avoir une zone de domination restreinte voire nulle ou quasiment nulle, dans la mesure où la zone entourant ledit joueur est considérée comme faisant partie de l'espace neutre.

La couleur blanche est généralement associée à ou aux espace(s) neutre(s) dans la cartographie.

La cartographie est ensuite affichée en temps réel au cours d'une étape 280 du procédé 200 sur un écran d'affichage à destination par exemple d'un entraîneur de l'une des deux équipes.

Avantageusement, la cartographie est synchronisée et superposée sur le flux vidéo acquis par la caméra 140.

Des indicateurs de performance peuvent également être affichés en temps réels concomitamment à la cartographie.

Il convient de souligner que les données de positionnement des joueurs de chaque équipe et de l'accessoire de jeu, ainsi que les cartographies affichées à chaque instant et les indicateurs de performances peuvent avantageusement être enregistrés pour analyser ou visionner après le match.

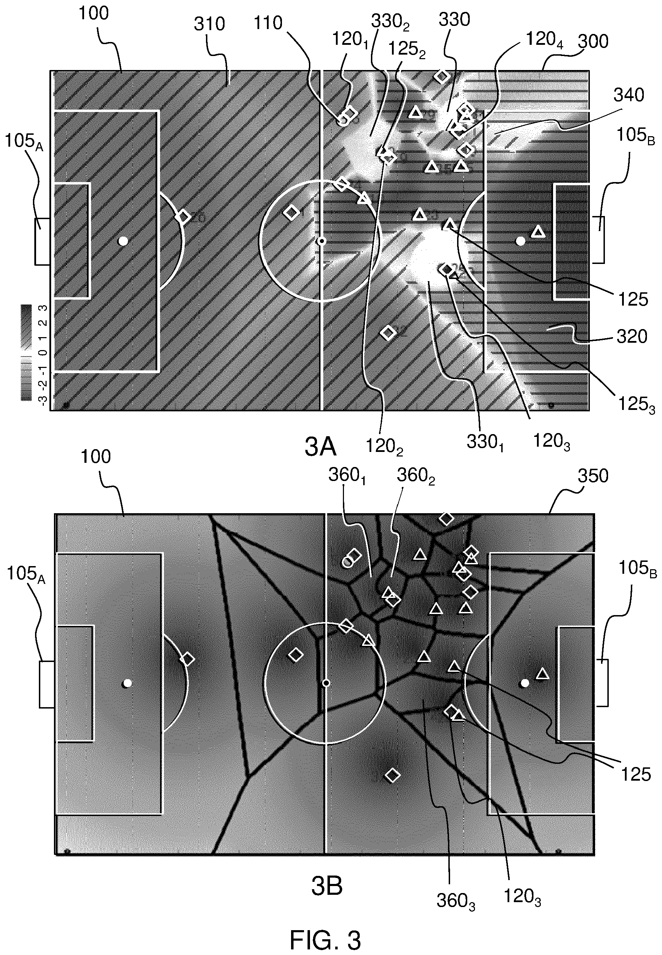

La figure 3 montre un exemple comparatif d'une cartographie 300 obtenue par le procédé 200 d'analyse (figure 3A) et d'une cartographie 350 obtenue par les méthodes de l'art antérieur (figure 3B). Les cartographies 300 et 350 sont calculées sur la phase de jeu illustrée en figure 1.

La cartographie 350 est partitionnée en partitions 360 par l'intermédiaire d'une méthode de l'art antérieur, tandis que l'intensité des couleurs représente le temps de parcours des différents joueurs.

Sur la cartographie 300, l'équipe A des joueurs 120 protégeant la zone de but $105_A$ et cherchant à envoyer le ballon 110 dans la zone de but $105_B$ est en position dominante sur la majeure partie du terrain 100. L'espace de domination 310 de l'équipe A est représentée par des hachures épaisses selon un angle de 45° par rapport à un bord du terrain 100.

L'équipe B des joueurs 125 protégeant la zone de but $105_B$ est dans une position défensive où l'ensemble des joueurs 125 sont dans le demi-terrain de droite sur la figure 3A. L'espace de domination 320 de l'équipe B est représentée sur la cartographie 300 par des hachures fines parallèles au bord longitudinal du terrain 100.

Sur la cartographie 300 obtenue par le procédé 200 d'analyse, sont présents des espaces neutres 330 où aucune équipe ne domine.

Généralement ces espaces neutres 330 correspondent à une position très proche d'au moins deux joueurs adverses, en d'autres termes d'au moins un joueur de chaque équipe. Ces espaces neutres, comme par exemple l'espace $330_2$, correspondent à un espace où aucun adversaire ne peut dominer car la différence entre les durées de déplacement des deux joueurs proches est très faible, inférieure au seuil prédéterminé. Il convient de souligner que l'espace $330_2$ correspond à deux partitions $360_1$ et $360_2$ dans la cartographie 350 obtenue avec les méthodes de l'art antérieur, où la partition $360_1$ est dominée par l'équipe A, tandis que la partition $360_2$ est dominée par l'équipe B. En outre, il convient également de souligner que l'intensité dans la cartographie 350 n'est pas fonction de la position des joueurs adversaires, mais simplement de la durée de déplacement du joueur associé à la partition.

Notamment, si le joueur $120_1$ envoie le ballon 110 dans l'espace $330_2$, en pensant que le joueur $120_2$ y serait notablement dominant, il n'est pas à exclure que son adversaire $125_2$ mette la pression sur le joueur $120_2$ et récupère le ballon, car le temps d'avance à l'arrivée du premier y sera quasi-nul, du fait de la proximité du joueur $125_2$.

En d'autres termes, sur la cartographie 300 obtenue par le procédé 200 d'analyse, des espaces neutres 330 où aucune équipe ne domine apparaissent, alors que dans la cartographie 350 obtenue avec les méthodes de l'art antérieur montre une zone de domination associée à un joueur de l'une des deux équipes.

Notamment, si le joueur $120_1$ envoie le ballon 110 dans l'espace $330_1$ en pensant que le joueur 120s y serait dominant, il n'est pas à exclure que son adversaire 125s puisse récupérer le ballon 110.

Dans la cartographie 350, il apparait que la partition $360_3$ comprise entre deux joueurs 125 est dominée par le joueur 120s de l'équipe A. Alors que dans la cartographie 300, cette partition 360s correspond à un espace neutre $330_1$ due à l'éloignement important du ballon 110.

Par ailleurs, la cartographie 300 montre une zone 340 de l'espace de domination 310, très favorable à l'équipe A. Cette zone de domination 340 du joueur $120_4$ correspond à l'action potentielle de jeu où le joueur $120_1$ envoie le ballon en direction du joueur $120_4$. En d'autres termes, le joueur $120_1$ passe le ballon 110 au joueur $120_4$, dans sa course.

Si la passe est effectuée correctement par le joueur $120_1$, le joueur $120_4$ peut alors se trouver en position favorable avec le ballon 110 dans la zone de domination 340 où il peut tenter de frapper dans le ballon 110 pour l'envoyer dans la zone de but $105_B$, ce qui peut aboutir à un marquage au score pour l'équipe A.

Il convient de souligner que la cartographie 300 permet d'apprécier cette situation très favorable à l'équipe A, en ce qu'elle révèle que la zone 340 est notablement dominée par le joueur $120_4$, ce qui n'est pas visible sur la cartographie 350 obtenue avec les méthodes de l'art antérieur, car les méthodes de l'art antérieur sont uniquement basées sur la position et la vitesse instantanée de chaque joueur 120 et 125, indépendamment de l'autre équipe, c'est-à-dire sans tenir compte de la position instantanée et/ou des trajectoires possibles des adversaires et/ou du ballon 110.

En outre, la cartographie 300 montre également des espaces neutres où aucune équipe n'est dominante, du fait de l'éloignement du ballon et/ou de la proximité d'un adversaire.

Il convient de souligner que la représentation du terrain 100 par la cartographie 300 est partitionnée intégralement en espaces qui correspondent soit à un espace de domination d'une des deux équipes, soit à un espace neutre.

Le procédé d'analyse 200 permet ainsi de caractériser quantitativement l'occupation dynamique du terrain 100, en effectuant des prédictions pertinentes de tous les déplacements physiquement possibles.

En résumé, le procédé 200 permet de révéler l'existence d'espaces neutres induits par la distance du ballon et de mettre en avant un avantage dynamique d'un joueur d'une équipe sur son adversaire direct, comme c'est le cas notamment du joueur $120_4$.

A partir de l'analyse effectuée par le procédé 200, il est également possible de déterminer un certain nombre d'indicateurs caractérisant chaque joueur, comme par exemple la qualité de passe effectuée par un joueur donné en tenant compte des zones de dominations disponibles de chaque joueur coéquipier, de la qualité de la domination dans chaque zone et des zones neutres.

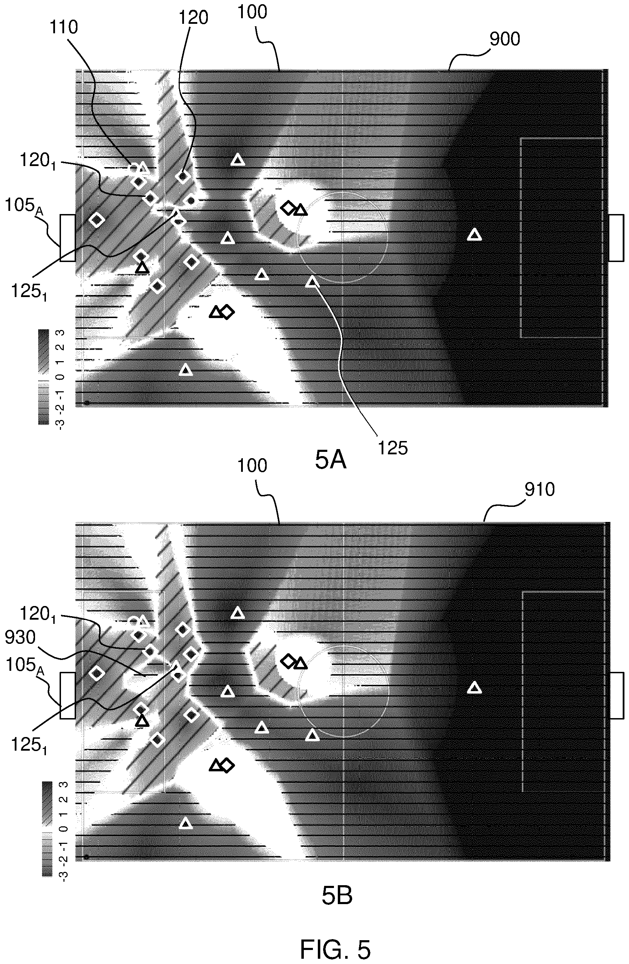

La figure 5 est une comparaison de deux cartographies obtenues par le procédé d'analyse 200. La cartographie 900 de la figure 5A est calculée à partir des positions instantanées et des vitesses instantanées des joueurs 120 et 125 acquises à un instant donné d'une phase de jeu. Tandis que la cartographie 910 de la figure 5B est calculée à partir des mêmes positions instantanées et vitesses instantanées des joueurs 120 et 125 acquises au même instant, sauf que la vitesse du joueur $125_1$ a été modifiée (passée de 3 km/h à 30 km/h) *a posteriori* par un individu tel que l'entraineur, par l'intermédiaire d'une interface de contrôle afin de voir l'impact sur la phase de jeu.

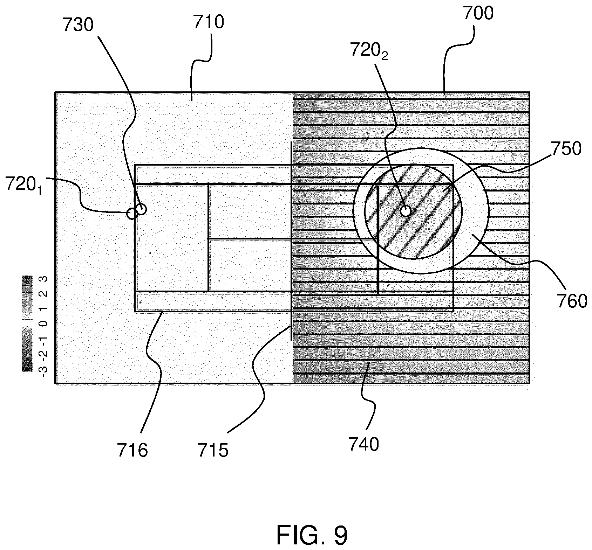

Une zone de domination 930 favorable à l'équipe B apparaît ainsi dans la figure 9B où le joueur $125_1$ aurait pu se retrouver dans une situation très favorable en face du but $105_A$ s'il avait déjà lancé une course vers l'avant, qui aurait pu aboutir à un marquage au score.

Autre exemple de mode de réalisation particulier

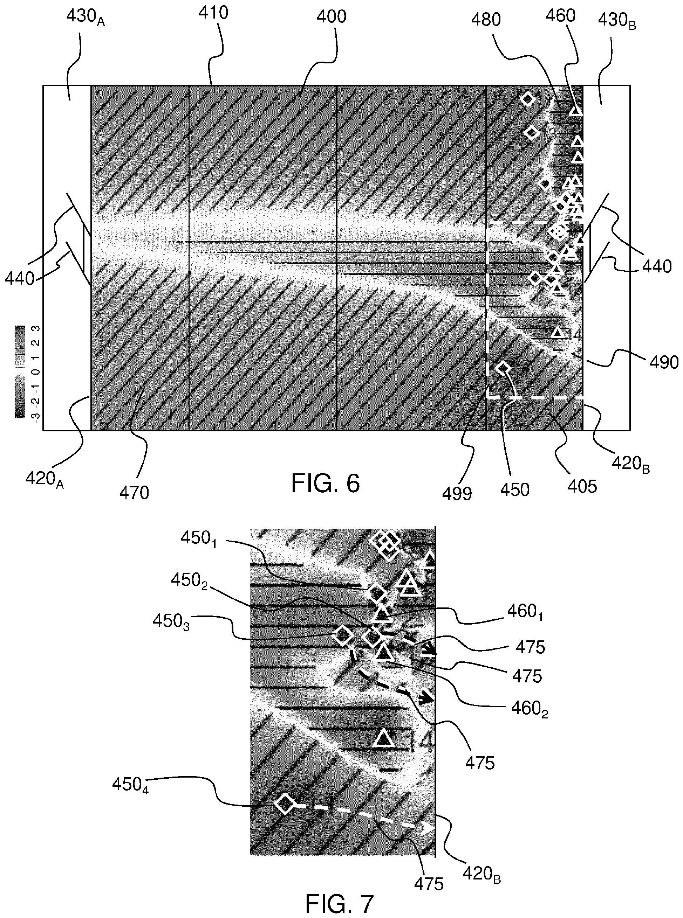

La figure 6 est une cartographie 400 obtenue par un procédé d'analyse d'une occupation dynamique d'un terrain selon l'invention. Il convient de souligner que le système d'analyse mettant en oeuvre le procédé d'analyse est similaire à celui illustré en figure 1.

Dans le présent exemple non limitatif de l'invention, la cartographie 400 correspond à une analyse de l'occupation dynamique d'un terrain 410 de rugby par deux équipes jouant au rugby à XV. L'accessoire de jeu est ici un ballon de rugby (non représenté sur la figure 6) qui a la particularité d'être de forme ovale.

Le but principal du jeu pour chaque équipe est de porter le ballon de rugby derrière la ligne 420 d'en-but défendue par l'équipe adverse, afin de toucher le sol dans une zone de but 430, couramment appelée l'en-but, avec le ballon et marquer ainsi les points d'un essai. Cette action correspondra par la suite à l'action de « marquer un essai ».

Un autre but du jeu est d'envoyer le ballon de rugby entre deux poteaux 440 situés sur la ligne 420 d'en but adverse, symétriquement par rapport au centre de la ligne d'en but. Cet autre but, correspondant au marquage d'un « drop », est généralement secondaire car moins rémunérateur de points qu'un essai.

En outre, il convient de souligner qu'une équipe marquant un essai a également la possibilité d'obtenir des points supplémentaires en « transformant l'essai » par une tentative de coup de pied arrêté.

Les joueurs 450 de l'équipe A, défendant l'en-but $430_A$ du terrain 410 sont représentés sur la figure 6 par des losanges. Les joueurs 460 de l'équipe B, défendant l'en-but $430_B$ du terrain 410 sont quant à eux représentés par des triangles.

La cartographie 400 est délimitée en au moins trois espaces :

- un espace 470 de domination de l'équipe A, correspondant aux zones hachurées en diagonale sur la cartographie 400 ;

- un espace 480 de domination de l'équipe B, correspondant aux zones hachurées horizontalement sur la cartographie 400 ; et

- un espace 490 neutre, correspond aux zones non hachurées sur la cartographie 400.

Il convient de souligner que l'espace 470 n'est ici pas continu.

L'espace neutre 490 correspond quant à lui à une zone tampon entre les deux espaces 470 et 480 de domination de chaque équipe.

L'occupation du terrain illustrée en figure 6, correspond à une phase où les deux équipes A et B sont dans la zone 405 du terrain 410, dite des « 22 mètres » de l'équipe B, à proximité de la ligne d'en-but $420_B$. L'occupation du terrain est ici favorable à l'équipe A qui accule l'équipe B à sa ligne d'en-but $420_B$.

La figure 7 est un agrandissement de la partie 499 de la cartographie 400.

En considérant que le ballon de rugby est dans les mains du joueur $450_1$, si le joueur $450_1$ réussit sa passe en direction du joueur $450_2$, le joueur $450_2$ a la possibilité de marquer un essai, en se faufilant entre les joueurs adverses $460_1$ et $460_2$ comme le montre la zone 471 de l'espace de domination 470.

Par contre, si le ballon est intercepté par le joueur adverse $460_1$, celui-ci a la possibilité de s'échapper en direction de l'en-but $430_A$ afin d'y marquer un essai pour l'équipe B.

Le joueur $450_1$ a également la possibilité de passer le ballon aux joueurs 450s et $450_4$ qui sont également dans des positions favorables pour marquer un essai.

En d'autres termes, l'espace de domination 470 montre trois chemins 475 allant jusqu'à la ligne d'en-but $420_B$ de l'équipe B, ces chemins 475 correspondant à des actions favorables pour l'équipe A. Si l'un de ces chemins 475 est suivi par un joueur 450, un essai est généralement marqué par l'équipe A.

Il convient de souligner que si le joueur $450_1$ passe le ballon à sa gauche, correspondant à la partie supérieure de l'espace de domination 470, l'équipe A ne serait pas en position favorable pour marquer un essai car les joueurs de l'équipe B sont bien positionnés en défense dans cette partie du terrain.

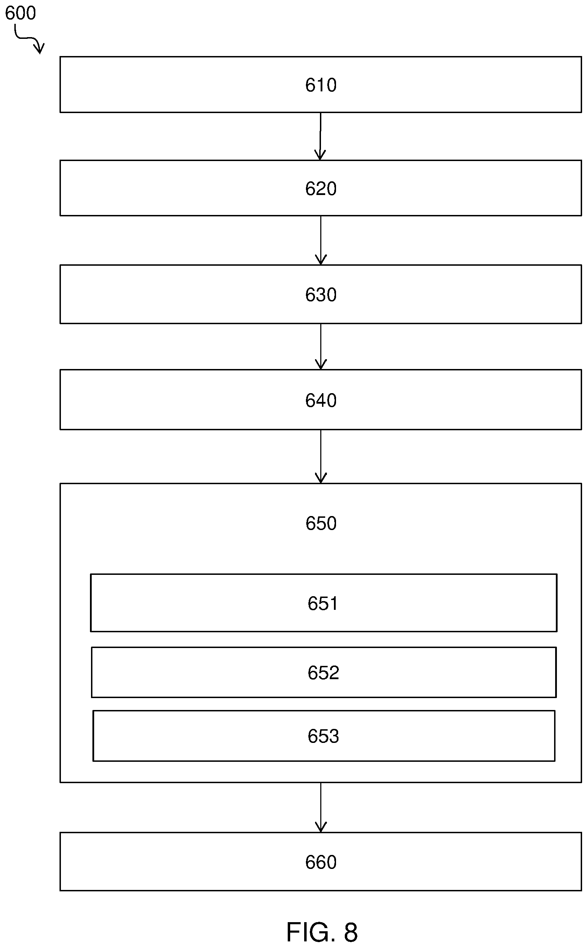

Le procédé 600 d'analyse de l'occupation dynamique du terrain, permettant d'obtenir la cartographie 400, est illustré sous la forme d'un schéma synoptique en figure 8.

Le procédé 600 d'analyse comprend une première étape 610 d'acquisition d'un flux vidéo par au moins trois caméras avantageusement fixes et acquérant chacune une partie du terrain 410 de rugby. Il convient de souligner que les images obtenues par chaque caméra se chevauchent en partie afin de pouvoir obtenir une image intégrale du terrain 410.

A partir d'une analyse des images intégrales du terrain 410, la position instantanée de chaque joueur 450 et 460 est obtenue au cours d'une deuxième étape 620 similaire à l'étape 240 du précédent mode de mise en oeuvre de l'invention.

Un vecteur vitesse instantanée associé à chaque joueur présent sur le terrain 410 est également calculé au cours d'une troisième étape 630 à partir de deux positions successives dudit joueur préalablement établies au cours de la deuxième étape 620.

La durée de déplacement entre la position instantanée de chaque joueur sur le terrain 410 et au moins un point du terrain 410 est déterminé au cours d'une quatrième étape 640 en fonction du vecteur vitesse instantanée et de paramètres physiques associés audit joueur. Ces paramètres physiques sont notamment une accélération caractéristique dudit joueur et une vitesse caractéristique dudit joueur.

Il convient de souligner que le calcul de la durée de déplacement peut être effectué seulement sur une partie du terrain, en fonction de la position des joueurs, afin d'optimiser le temps de calcul.

Le procédé 600 comprend ensuite une cinquième étape 650 générant la cartographie 400 représentative de l'occupation dynamique du terrain 410 par les deux équipes.

Le terrain 410 est délimité au cours de cette étape 650 en trois espaces : un espace de domination pour chaque équipe et un espace neutre. Il convient de souligner que chaque espace peut être discontinu ou disjoint.

L'espace de domination de chaque équipe, à savoir l'espace 470 pour l'équipe A et l'espace 480 pour l'équipe B, est calculé au cours d'une sous-étape 651 de l'étape 650.

L'espace de domination de chaque équipe correspond à l'ensemble des points du terrain 410 pour lesquels un joueur de ladite équipe a un temps d'avance par rapport aux joueurs de l'équipe adverse supérieur à un seuil prédéterminé, le temps d'avance étant égal à la différence entre la durée de déplacement de deux joueurs vers le même point.

L'espace neutre est déterminé au cours d'une sous-étape 652 de l'étape 650 et correspond à l'ensemble des points du terrain pour lesquels la différence entre la durée minimale de déplacement de chaque équipe est inférieure en valeur absolue au seuil prédéterminé (par exemple de 0,1 s), la durée minimale de déplacement de chaque équipe vers un point du terrain correspondant à la valeur minimale des durées de déplacement des joueurs de ladite équipe vers ce point.

En d'autres termes, la valeur du seuil prédéterminé permet de délimiter les limites des trois espaces.

Généralement, chaque espace est associé à une couleur distincte, telle que bleue pour l'espace 470, rouge pour l'espace 480 et blanc pour l'espace neutre 490.

L'étape 650 comprend également dans le présent exemple non limitatif de l'invention, une sous-étape 653 de détermination d'une intensité de couleur des différents espaces, l'intensité étant fonction du maximum de temps d'avance des joueurs d'une équipe par rapport aux joueurs de l'équipe adverse. Plus le temps d'avance est important, plus l'intensité est élevée.

Il convient de souligner que les valeurs des intensités de couleur de la cartographie forment une surface continue, dont les gradients de couleur sont également continus.

La cartographie 400 obtenue, ainsi que des indicateurs de performance, sont ensuite affichés en temps réel au cours d'une sixième étape 660 du procédé 600, sur un écran d'affichage.

Autre exemple de mode de réalisation particulier

La figure 9 représente une cartographie 700 d'une occupation dynamique d'un terrain 710 de tennis, obtenue par un procédé similaire à celui illustré en figure 2 ou en figure 8. Le système d'analyse mettant en oeuvre le procédé d'analyse est similaire à celui illustré en figure 1.

La cartographie 700 est dans le présent exemple non limitatif de l'invention calculée sur la moitié du terrain 710 délimité par un filet 715. La moitié du terrain 710 considérée correspond sur la figure 9 à la moitié située à droite du filet 715.

Il convient de souligner que le calcul n'est pas restreint à l'intérieur du marquage 716 du terrain 710 de tennis, mais effectué également à l'intérieur d'une bande en pourtour du marquage 716.

Deux joueurs 720 s'affrontent dans un match dit de « simple » et échangent une balle 730 qui est un accessoire de jeu mobile. Au cours d'un jeu, le point est généralement marqué par un joueur lorsque le joueur adverse commet une faute en renvoyant la balle. Une faute est par exemple commise lorsque le premier rebond de la balle après le franchissement du filet est effectué en dehors des marquages du terrain de simple ou lorsque le joueur renvoie la balle après deux rebonds dans sa moitié de terrain.

Dans la phase de jeu illustrée en figure 9, la balle 730 est détectée, ou considérée, au niveau du joueur $720_1$ située à gauche du filet 715 sur la cartographie 700, qui va chercher à la renvoyer dans la moitié de terrain adverse occupée par le joueur $720_2$.

Il convient de souligner que dans le présent exemple non limitatif de l'invention, les deux joueurs 720 forment chacun une équipe distincte. En d'autres termes, chaque équipe comprend un seul joueur.

La détermination de l'occupation dynamique du terrain est effectuée ici sur la moitié du terrain du joueur $720_2$.

La cartographie 700 est ainsi délimitée en trois espaces :

- un espace 740 de domination du joueur $720_1$ représenté sur la figure 9 par des hachures horizontales ;

- un espace 750 de domination du joueur $720_2$ représenté sur la figure 9 par des hachures en diagonale ;
- un espace 760 neutre où aucun joueur n'est considéré comme dominant.

Il convient de souligner que les trois espaces sont calculés en fonction d'une trajectoire possible de la balle 730 et sont représentatifs de la possibilité de renvoyer la balle par le joueur $720_2$.

Par exemple, si la balle 730 est renvoyée en direction de l'espace 750, le joueur $720_2$ n'aura aucun mal à renvoyer la balle et à mettre le joueur $720_1$ en difficulté. Le joueur $720_2$ aura par conséquent plus de probabilité de marquer au score, en réalisant un coup gagnant.

Au contraire, si la balle 730 est renvoyée dans l'espace 740, le joueur $720_2$ sera *a priori* en difficulté pour renvoyer la balle 730, voire ne pourra pas la renvoyer, ce qui augmente la probabilité pour le joueur $720_1$ de marquer au score. Le joueur $720_1$ réalise ainsi un coup gagnant.

Si la balle arrive dans l'espace 760, aucun des joueurs ne dominant, l'échange devrait *a priori* se poursuivre.

Il est ainsi possible de repérer où il faut envoyer la balle 730 pour réaliser un coup gagnant lorsqu'une situation favorable se présente pour l'un des joueurs 720.

Des indicateurs de performance peuvent également être déterminés concomitamment avec la cartographie 700. De tels indicateurs sont par exemple :

- la qualité de la couverture du terrain avant le coup de l'adversaire ;
- le nombre de fautes directes, estimées par les actions où la balle n'est pas correctement renvoyée bien qu'elle ait été réceptionnée par un joueur dans son espace de domination ;
- la qualité de chaque renvoi de la balle ;
- les opportunités de renvois disponibles à chaque instant ou sur tout ou partie du match.

Autres avantages et caractéristiques optionnelles

Dans des variantes des modes de réalisation précédents, la cartographie est délimitée en au moins quatre espaces, dont un espace de domination pour chaque, un espace neutre et un espace quasi-neutre, afin de mettre en avant les espaces faiblement dominés par l'une des deux équipes.

Il convient de souligner que la subdivision est généralement fonction d'une comparaison entre la durée de déplacement d'au moins un joueur de chaque équipe, voire de la durée de déplacement de l'accessoire de jeu.

Dans des variantes des modes de réalisation précédents, le système d'analyse comprend des moyens d'interaction permettant à un individu de modifier tout ou partie des données associées à un élément mobile, comme par exemple la position instantanée de l'élément mobile ou la vitesse instantanée de l'élément mobile afin de voir l'impact sur la phase de jeu en cours d'analyse. En outre, le système d'analyse peut être utilisé sur des données de positions et de vitesses instantanées, de tout ou partie des éléments mobiles, définies selon un scénario préétabli par un entraîneur afin de tester différentes tactiques au regard d'au moins une phase de jeu.

**Revendications**

1. Procédé (200 ; 600) d'analyse d'une occupation dynamique d'un terrain (100 ; 410 ; 710) par une pluralité d'éléments mobiles, un élément mobile étant un joueur d'une équipe parmi deux équipes adversaires d'un jeu ou un accessoire de jeu (110 ; 730), tel qu'un ballon, une balle, un palet ou un volant, chaque équipe comprenant au moins un joueur (120, 125 ; 450, 460 ; 720), le procédé étant mis en oeuvre par un système (130) d'analyse d'une occupation dynamique comprenant des moyens d'acquisition, des moyens de détermination et des moyens de délimitation, le procédé comportant des étapes de :

   - acquisition (240 ; 620) par des moyens d'acquisition d'une position instantanée pour tout ou partie des éléments mobiles sur le terrain à au moins deux instants distincts ;
   - pour chaque élément mobile, détermination (250 ; 630) d'un vecteur vitesse instantanée ;
   - pour chaque élément mobile localisé, détermination (260 ; 640) d'une durée de déplacement entre la position instantanée dudit élément mobile et tout ou partie des points du terrain en fonction dudit vecteur vitesse instantanée, et d'un modèle de déplacement associé audit élément mobile ;
   - pour chaque élément mobile localisé et pour tout ou partie des points du terrain, détermination par des moyens de détermination du temps d'avance dudit élément mobile en ledit point du terrain, ledit temps d'avance étant calculé par une comparaison entre la durée de déplacement dudit élément mobile vers ledit point du terrain et la durée de déplacement d'un autre élément mobile vers le même point ;
   - pour tout ou partie des joueurs localisés, délimitation par des moyens de délimitation d'une zone sur le terrain, dite zone de domination dudit joueur, correspondant à l'ensemble des points du terrain pour lesquels le temps d'avance dudit joueur est supérieur à un seuil prédéterminé.

2. Procédé selon la revendication 1, comprenant également une étape de délimitation du terrain en trois espaces : un espace de domination (310, 320 ; 470, 480 ; 740, 750) pour chaque équipe et un espace neutre (330 ; 490 ; 760), l'espace de domination d'une équipe comprenant l'ensemble des zones de domination des joueurs de l'équipe, l'espace neutre correspondant à l'ensemble des points de tout ou partie du terrain non couverts par un espace de domination d'une équipe.

3. Procédé selon l'une quelconque des revendications 1 à 2, dans lequel l'espace de domination d'un joueur d'une équipe correspond à l'ensemble des points du terrain pour lesquels ledit joueur a un temps d'avance par rapport aux joueurs de l'équipe adverse supérieur à un seuil prédéterminé, le temps d'avance entre deux joueurs étant égal à la différence entre la durée de déplacement de deux joueurs vers le même point.

4. Procédé selon l'une quelconque des revendications 2 à 3, dans lequel l'espace neutre comprend les points de tout ou partie du terrain où la durée de déplacement de l'accessoire de jeu est supérieure à la durée de déplacement d'au moins un joueur de chaque équipe.

5. Procédé selon l'une quelconque des revendications 1 à 4, dans lequel une zone de domination d'un joueur d'une équipe correspond à l'ensemble des points du terrain pour lesquels à la fois :

   - la durée de déplacement dudit joueur vers un point dudit espace de domination est égale à la plus petite de toutes les durées de déplacement de tous les joueurs vers ce point, et
   - la durée de déplacement de l'accessoire de jeu vers le même point dudit espace de domination est inférieure à la plus petite des durées de déplacement des joueurs de l'autre équipe.

6. Procédé selon l'une quelconque des revendications 1 à 5, dans lequel les éléments mobiles dont les positions instantanées sont acquises sont uniquement des joueurs, le procédé comprenant également une étape de détermination de la position instantanée de l'accessoire de jeu comme étant égale à la position d'un joueur.

7. Procédé selon l'une quelconque des revendications 1 et 6, dans lequel le terrain comprend deux parties de terrain, les déplacements de chaque équipe étant contraints à l'intérieur d'une partie distincte du terrain, et dans lequel le temps d'avance d'un joueur est calculé par rapport aux joueurs de son équipe et à l'accessoire de jeu, le temps d'avance étant égal à la différence entre la durée de déplacement de l'accessoire de jeu vers ledit point et la durée de déplacement dudit joueur vers ce point.

8. Procédé selon la revendication 7, dans lequel le temps d'avance d'une équipe en un point de la partie de terrain occupée par ladite équipe correspond au maximum des temps d'avance des joueurs de ladite équipe.

9. Procédé selon l'une quelconque des revendications 7 à 8, dans lequel l'espace de domination d'une équipe dans la partie du terrain occupée par l'équipe adverse correspond à l'ensemble des points de ladite partie du terrain pour lesquels le temps d'avance de l'accessoire de jeu vers ledit point est supérieur à un seuil prédéterminé, le temps d'avance de l'accessoire de jeu étant égal à la différence entre la plus petite des durées de déplacement des joueurs de l'équipe adverse et la durée de déplacement de l'accessoire de jeu.

10. Procédé selon l'une quelconque des revendications 1 à 9, comprenant également une étape de génération (270 ; 650) d'une cartographie (300 ; 400 ; 700) représentative de l'occupation du terrain par chaque équipe.

11. Procédé selon la revendication 10, dans lequel l'étape de génération de la cartographie comprend une sous-étape (272 ; 653) de détermination d'une intensité de couleur fonction de la domination du joueur, chaque équipe étant représentée par une couleur distincte.

12. Procédé selon l'une quelconque des revendications 10 à 11, dans lequel la génération de la cartographie est effectuée en temps réel.

13. Procédé selon l'une quelconque des revendications 1 à 12, dans lequel le procédé comprend également une étape d'acquisition (210 ; 610) d'un flux vidéo par au moins une caméra (140) couvrant tout ou partie du terrain, et dans lequel l'étape d'acquisition de la position instantanée de l'accessoire de jeu comprend une sous-étape d'analyse du flux vidéo pour déterminer ladite position instantanée.

14. Procédé selon la revendication 13, dans lequel l'étape d'acquisition de la position instantanée de chaque joueur comprend une sous-étape d'analyse du flux vidéo pour déterminer ladite position instantanée.

15. Procédé selon l'une quelconque des revendications 13 à 14, dans lequel la cartographie est synchronisée et superposée sur le flux vidéo.

16. Procédé selon l'une quelconque des revendications 13 à 15, dans lequel le procédé comprend également une étape de suivi de tout ou partie des joueurs et/ou de l'accessoire de jeu dans le flux vidéo.

17. Procédé selon l'une quelconque des revendications 13 à 16, dans lequel le procédé comprend également des étapes de :

- affichage en temps réel du flux vidéo sur lequel est superposé au moins un indicateur de suivi d'un joueur ; et
- repositionnement de l'indicateur de suivi par un opérateur.

18. Procédé selon l'une quelconque des revendications 1 à 17, dans lequel l'étape d'acquisition de la position instantanée de chaque joueur et/ou de l'accessoire de jeu sur le terrain comprend une sous-étape d'analyse d'un signal émis par une balise solidarisée audit joueur et/ou audit accessoire de jeu et reçu par au moins une borne située à proximité du terrain.

19. Procédé selon l'une quelconque des revendications 1 à 18, dans lequel le procédé comprend également une étape de détermination d'au moins un indicateur quantitatif d'une caractéristique qualitative d'un joueur choisie parmi :

- une valeur représentative de la domination du joueur dans au moins une partie du terrain, calculée à partir des caractéristiques des zones de domination enregistrées au cours d'un match ;
- une qualité de passe de l'accessoire de jeu ;
- une valeur représentative de la pression subie par ledit joueur ;
- une qualité de frappe dans l'accessoire de jeu ;
- un taux de fautes directes.

20. Système (130) d'analyse d'une occupation dynamique d'un terrain (100) par deux équipes adversaires d'un jeu, mettant en oeuvre le procédé d'analyse selon l'une quelconque des revendications 1 à 19, comprenant :

- des moyens informatiques de traitement de données comportant un processeur et une mémoire informatique ;
- des moyens d'acquisition de la position instantanée d'un élément mobile sur le terrain, l'élément mobile étant un joueur ou l'accessoire de jeu ;
- des moyens de détermination d'une valeur représentative du temps d'avance d'un élément mobile en un point du terrain par rapport à un autre élément mobile ;
- des moyens de délimitation d'une zone sur le terrain pour un joueur, dite zone de domination dudit joueur, ladite zone correspondant à l'ensemble des points pour lesquels ledit joueur a un temps d'avance supérieur à un seuil prédéterminé.

21. Système selon la revendication 20, dans lequel les moyens d'acquisition de la position instantanée de chaque élément mobile comprennent au moins une caméra (140) acquérant un flux vidéo couvrant tout ou partie du terrain, et/ou au moins une balise solidarisée audit élément mobile et au moins une borne de réception d'un signal émis par ladite balise.

22. Système selon l'une quelconque des revendications 20 à 21, comprenant également des moyens de génération et d'affichage d'une cartographie représentative de l'occupation dynamique du terrain.

23. Système selon l'une quelconque des revendications 20 à 22, comprenant également des moyens d'enregistrement des données acquises et de modification par un utilisateur de tout ou partie des données enregistrées de positions instantanées et/ou vitesses des éléments mobiles.

**Patentansprüche**

1. Verfahren (200; 600) zum Analysieren einer dynamischen Belegung eines Sportfelds (100; 410; 710) durch eine Vielzahl von beweglichen Elementen, wobei ein bewegliches Element jeweils ein Spieler einer von zwei gegnerischen Mannschaften eines Spiels oder ein Spielgerät (110; 730), wie etwa ein Ball, eine Kugel, ein Puck oder ein Federball, ist, wobei jede Mannschaft mindestens einen Spieler (120, 125; 450, 460; 720) umfasst, wobei das Verfahren durch ein System (130) zum Analysieren einer dynamischen Belegung, das Erfassungsmittel, Bestimmungsmittel und Begrenzungsmittel umfasst, durchgeführt wird, wobei das Verfahren Folgendes umfasst:

   - Erfassen (240; 620) einer momentanen Position für alle oder einige der beweglichen Elemente auf dem Sportfeld zu mindestens zwei unterschiedlichen Zeitpunkten durch die Erfassungsmittel;
   - Bestimmen (250; 630), für jedes bewegliche Element, eines momentanen Geschwindigkeitsvektors;
   - Bestimmen (260; 640), für jedes lokalisierte bewegliche Element, einer Bewegungszeit zwischen der momentanen Position des beweglichen Elements und allen oder einem Teil der Punkte auf dem Sportfeld in Abhängigkeit von dem momentanen Geschwindigkeitsvektor und eines mit dem beweglichen Element verbundenen Bewegungsmodells;
   - für jedes lokalisierte bewegliche Element und für alle oder einen Teil der Punkte des Sportfelds, Bestimmen einer kommenden Zeit des beweglichen Elements an dem Punkt des Sportfelds durch die Bestimmungsmittel, wobei die kommende Zeit durch einen Vergleich zwischen der Bewegungsdauer des beweglichen Elements in Richtung des Punkts des Sportfelds und der Bewegungsdauer eines anderen beweglichen Elements in Richtung desselben Punkts berechnet wird;
   - für alle oder einen Teil der lokalisierten Spieler, Begrenzen, durch die Begrenzungsmittel, einer Zone auf dem Sportfeld, der sogenannte Dominanzzone des Spielers, der dem Satz der Punkte des Spielfelds entspricht, für die die kommende Zeit des Spielers größer als ein vorbestimmter Schwellenwert ist.

2. Verfahren nach Anspruch 1, das außerdem einen Schritt des Begrenzens des Sportfelds in drei Bereiche umfasst: einen Dominanzbereich (310, 320; 470, 480; 740, 750) für jede Mannschaft und einen neutralen Bereich (330; 490; 760), wobei der Dominanzbereich einer Mannschaft alle Dominanzbereiche der Spieler der Mannschaft umfasst, wobei der neutrale Bereich dem Satz der Punkte des gesamten oder eines Teils des Sportfelds entspricht, die nicht von einem Dominanzbereich einer Mannschaft abgedeckt werden.

3. Verfahren nach einem der Ansprüche 1 bis 2, wobei der Dominanzbereich eines Spielers einer Mannschaft dem Satz der Punkte des Sportfelds entspricht, für die der Spieler eine kommende Zeit im Verhältnis zu den Spielern der gegnerischen Mannschaft hat, der größer als ein vorbestimmter Schwellenwert ist, wobei die kommende Zeit zwischen zwei Spielern gleich der Differenz zwischen der Bewegungsdauer zweier Spieler in Richtung desselben Punkts ist.

4. Verfahren nach einem der Ansprüche 2 bis 3, wobei der neutrale Bereich die Punkte des gesamten oder eines Teils des Sportfelds umfasst, an denen die Bewegungsdauer des Spielgeräts größer ist als die Bewegungsdauer mindestens eines Spielers jeder Mannschaft.

5. Verfahren nach einem der Ansprüche 1 bis 4, wobei ein Dominanzbereich eines Spielers einer Mannschaft einem Satz der Punkte des Sportfelds entspricht, bei denen zugleich:

   - die Bewegungsdauer des Spielers zu einem Punkt des Dominanzbereichs gleich der kleinsten aller Bewegungsdauern aller Spieler zu diesem Punkt ist, und
   - die Bewegungsdauer des Spielgeräts zum gleichen Punkt des Dominanzbereichs kleiner als die kleinste Bewegungsdauer der Spieler der anderen Mannschaft ist.

6. Verfahren nach einem der Ansprüche 1 bis 5, wobei die beweglichen Elementen, deren momentane Positionen erfasst werden, nur Spieler sind, wobei das Verfahren außerdem einen Schritt des Bestimmens der momentanen Position des Spielgeräts als gleich der Position eines Spielers umfasst.

7. Verfahren nach einem der Ansprüche 1 und 6, wobei das Sportfeld zwei Sportfeldabschnitte umfasst, wobei die Bewegungen jeder Mannschaft auf ein Inneres eines separaten Abschnitts des Sportfelds beschränkt sind, und wobei die kommende Zeit eines Spielers im Verhältnis zu den Spielern seiner Mannschaft und dem Spielgerät berechnet wird, wobei die kommende Zeit gleich der Differenz zwischen der Bewegungsdauer des Spielgeräts in Richtung des Punkts und der Bewegungsdauer des Spielers in Richtung dieses Punkts ist.

8. Verfahren nach Anspruch 7, wobei die kommende Zeit einer Mannschaft an einem Punkt des von der Mannschaft belegten Sportfeldabschnitts der maximalen kommenden Zeit der Spieler der Mannschaft entspricht.

9. Verfahren nach einem der Ansprüche 7 bis 8, wobei der Dominanzbereich einer Mannschaft in einem von der gegnerischen Mannschaft belegten Sportfeldabschnitt einem Satz der Punkte des Teils des Sportfelds entspricht, für die die kommende Zeit des Spielgeräts größer als ein vorbestimmter Schwellenwert ist, wobei die kommende Zeit des Spielgeräts gleich der Differenz zwischen der kleinsten der Bewegungsdauern der Spieler der gegnerischen Mannschaft und der Bewegungsdauer des Spielgeräts ist.

10. Verfahren nach einem der Ansprüche 1 bis 9, das außerdem einen Schritt des Erzeugens (270; 650) einer Karte (300; 400; 700) umfasst, die die Belegung des Sportfelds durch jede Mannschaft darstellt.

11. Verfahren nach Anspruch 10, wobei der Schritt des Erzeugens der Karte einen Unterschritt (272; 653) des Bestimmens einer Farbintensität in Abhängigkeit von der Dominanz des Spielers umfasst, wobei jede Mannschaft durch eine bestimmte Farbe dargestellt wird.

12. Verfahren nach einem der Ansprüche 10 bis 11, wobei das Erzeugen der Karte in Echtzeit ausgeführt wird.

13. Verfahren nach einem der Ansprüche 1 bis 12, wobei das Verfahren außerdem einen Schritt des Erfassens (210; 610) eines Videostreams durch mindestens eine Kamera (140), der das gesamte oder einen Teil des Sportfelds abdeckt, umfasst, und wobei der Schritt des Erfassens der momentanen Position des Spielgeräts einen Unterschritt des Analysierens des Videostreams umfasst, um die momentane Position zu bestimmen.

14. Verfahren nach Anspruch 13, wobei der Schritt des Erfassens der momentanen Position jedes Spielers einen Unterschritt des Analysierens des Videostreams umfasst, um die momentane Position zu bestimmen.

15. Verfahren nach einem der Ansprüche 13 bis 14, wobei die Karte synchronisiert und über den Videostream gelegt wird.

16. Verfahren nach einem der Ansprüche 13 bis 15, wobei das Verfahren außerdem einen Schritt des Verfolgens aller oder eines Teils der Spieler und/oder des Spielgeräts im Videostream umfasst.

17. Verfahren nach einem der Ansprüche 13 bis 16, wobei das Verfahren außerdem die folgenden Schritte umfasst:

   - Anzeigen des Videostreams in Echtzeit, über den mindestens ein Spieler-Verfolgungsindikator gelegt wird; und
   - Neupositionieren des Verfolgungsindikators durch einen Benutzer.

18. Verfahren nach einem der Ansprüche 1 bis 17, wobei der Schritt des Erfassens der momentanen Position jedes Spielers und/oder des Spielgeräts auf dem Sportfeld einen Unterschritt des Analysierens eines Signals umfasst, das von einem am Spieler und/oder am Spielgerät angebrachten Ortungsgerät ausgesendet und von mindestens einem Endgerät, das sich in der Nähe des Sportfelds befindet, empfangen wird.

19. Verfahren nach einem der Ansprüche 1 bis 18, wobei das Verfahren außerdem einen Schritt des Bestimmens mindestens eines quantitativen Indikators einer qualitativen Eigenschaft eines Spielers umfasst, ausgewählt aus:

   - einem Wert, der die Dominanz des Spielers in mindestens einem Abschnitt des Sportfelds darstellt und der aus den während eines Spiels aufgezeichneten Eigenschaften der Dominanzbereiche berechnet wird;
   - einer Passqualität des Spielgeräts;
   - einem Wert, der den vom Spieler erfahrenen Druck darstellt;
   - einer Schlagqualität des Spielgeräts;
   - einer direkte Fehlerquote.

20. System (130) zum Analysieren einer dynamischen Belegung eines Sportfelds (100) durch zwei gegnerische Mannschaften eines Spiels, das das Analyseverfahren nach einem der Ansprüche 1 bis 19 durchführt und Folgendes umfasst:

- Computermittel zum Verarbeiten von Daten, die einen Prozessor und einen Computerspeicher umfassen;
- Mittel zum Erfassen der momentanen Position eines beweglichen Elements auf dem Sportfeld, wobei das bewegliche Element ein Spieler oder das Spielgerät ist;
- Mittel zum Bestimmen eines Werts, der eine kommende Zeit eines beweglichen Elements an einem Punkt des Sportfelds im Verhältnis zu einem anderen beweglichen Element darstellt;
- Mittel zum Begrenzen eines Bereichs auf dem Sportfeld für einen Spieler, den sogenannten Dominanzbereich des Spielers, wobei der Bereich einem Satz der Punkte entspricht, für die der Spieler eine kommende Zeit hat, die größer als ein vorbestimmter Schwellenwert ist.

21. System nach Anspruch 20, wobei die Mittel zum Erfassen der momentanen Position jedes beweglichen Elements mindestens eine Kamera (140), die einen Videostream erfasst, der das gesamte oder einen Teil des Sportfelds abdeckt, und/oder mindestens ein am beweglichen Element angebrachtes Ortungsgerät und mindestens ein Empfangsendgerät für das von dem Ortungsgerät ausgesendete Signal umfasst.

22. System nach einem der Ansprüche 20 bis 21, das außerdem Mittel zum Erzeugen und Anzeigen einer Karte umfasst, die die dynamische Belegung des Sportfelds darstellt.

23. System nach einem der Ansprüche 20 bis 22, das außerdem Mittel zum Aufzeichnen der erfassten Daten und zum Modifizieren aller oder eines Teils der aufgezeichneten Daten der momentanen Positionen und/oder Geschwindigkeiten der beweglichen Elemente durch einen Benutzer umfasst.

**Claims**

1. A method (200; 600) for analysing a dynamic occupation of a field (100; 410; 710) by a plurality of mobile elements, a mobile element being a player of one team among two opposing teams of a game or a game accessory (110; 730), such as a ball, a puck or a shuttlecock, each team comprising at least one player (120, 125; 450, 460; 720), the method being implemented by a system (130) for analysing a dynamic occupation comprising acquisition means, determination means and delimitation means, the method including steps of:

- acquiring (240; 620) by acquisition means an instantaneous position for all or part of the mobile elements on the field at least at two distinct times;
- for each mobile element, determining (250; 630) an instantaneous speed vector;
- for each located mobile element, determining (260; 640) a movement duration between the instantaneous position of said mobile element and all or part of the points on the field according to said instantaneous speed vector, and a movement model associated with said mobile element;
- for each located movable element and for all or part of the points on the field, determining, by determination means, the time of advance of said mobile element at said point on the field, said advance time being calculated by a comparison between the duration of movement of said mobile element towards said point on the field and the duration of movement of another mobile element towards the same point;
- for all or part of the located players, delimiting, by delimitation means, a zone on the field, called the domination zone of said player, corresponding to all the points on the field for which the lead time of said player is greater than a predetermined threshold.

2. The method according to claim 1, also comprising a step of delimiting the field into three spaces: a domination space (310, 320; 470, 480; 740, 750) for each team and a neutral space (330; 490; 760), the domination space of a team comprising all the domination zones of the team's players, the neutral space corresponding to all the points of all or part of the field not covered by a domination space of a team.

3. The method according to any one of claims 1 to 2, wherein the domination space of a player of a team corresponds to all the points on the field for which said player has a time lead compared to the players of the opposing team greater than a predetermined threshold, the time of advance between two players being equal to the difference between the duration of movement of two players towards the same point.

4. The method according to any one of claims 2 to 3, wherein the neutral space comprises the points of all or part of the field where the duration of movement of the game accessory is greater than the duration of movement of at least one player from each team.

5. The method according to any one of claims 1 to 4, wherein a zone of domination of a player of a team corresponds to all the points on the field for which both:

- the duration of movement of said player towards a point of said domination space is equal to the smallest of all the durations of movement of all the players towards this point, and

- the duration of movement of the game accessory towards the same point of said domination space is less than the shortest of the durations of movement of the players of the other team.

6. The method according to any one of claims 1 to 5, wherein the mobile elements whose instantaneous positions are acquired are only players, the method also comprising a step of determining the instantaneous position of the game accessory as being equal to a player's position.

7. The method according to any one of claims 1 and 6, wherein the field comprises two field parts, the movements of each team being constrained within a distinct part of the field, and wherein the time of advance of a player is calculated in relation to the players of his team and to the game accessory, the advance time being equal to the difference between the duration of movement of the game accessory towards said point and the duration of movement of said player towards this point.

8. The method according to claim 7, wherein the advance time of a team at a point of the part of the field occupied by said team corresponds to the maximum advance times of the players of said team.

9. The method according to any one of claims 7 to 8, wherein the domination space of a team in the part of the field occupied by the opposing team corresponds to all the points of said part of the field for which the advance time of the game accessory towards said point is greater than a predetermined threshold, the advance time of the game accessory being equal to the difference between the smallest of the movement times of the players of the opposing team and the duration of movement of the game accessory.

10. The method according to any one of claims 1 to 9, also comprising a step of generating (270; 650) a map (300; 400; 700) representative of the occupation of the field by each team.

11. The method according to claim 10, wherein the step of generating the map comprises a sub-step (272; 653) of determining a colour intensity depending on the dominance of the player, each team being represented by a distinct colour.

12. The method according to any one of claims 10 to 11, wherein the generation of the map is carried out in real time.

13. The method according to any one of claims 1 to 12, wherein the method also comprises a step of acquiring (210; 610) a video stream by at least one camera (140) covering all or part of the field, and wherein the step of acquiring the instantaneous position of the game accessory comprises a sub-step of analysing the video stream to determine said instantaneous position.

14. The method according to claim 13, wherein the step of acquiring the instantaneous position of each player comprises a sub-step of analysing the video stream to determine said instantaneous position.

15. The method according to any one of claims 13 to 14, wherein the map is synchronised and superimposed on the video stream.

16. The method according to any one of claims 13 to 15, wherein the method also comprises a step of tracking all or part of the players and/or the game accessory in the video stream.

17. The method according to any one of claims 13 to 16, wherein the method also comprises steps of:

    - displaying in real time the video stream on which at least one player tracking indicator is superimposed; and
    - repositioning the tracking indicator by an operator.

18. The method according to any one of claims 1 to 17, wherein the step of acquiring the instantaneous position of each player and/or of the game accessory on the field comprises a sub-step of analysing a signal emitted by a beacon secured to said player and/or said game accessory and received by at least one terminal located near the field.

19. The method according to any one of claims 1 to 18, wherein the method also comprises a step of determining at least one quantitative indicator of a qualitative feature of a player selected from:

    - a value representative of the domination of the player in at least part of the field, calculated from the features of the domination zones recorded during a match;
    - passing quality of the game accessory;
    - a value representative of the pressure experienced by said player;
    - hitting quality in the game accessory;
    - a rate of direct errors.

20. A system (130) for analysing a dynamic occupation of a field (100) by two opposing teams in a game, implementing the analysis method according to any one of claims 1 to 19, comprising:

- computer data processing means including a processor and a computer memory;
- means for acquiring the instantaneous position of a mobile element on the field, the mobile element being a player or the game accessory;
- means for determining a value representative of the advance time of a mobile element at a point on the field relative to another mobile element;
- means for delimiting a zone on the field for a player, called the domination zone of said player, said zone corresponding to all the points for which said player has a lead time greater than a predetermined threshold.

21. A system according to claim 20, wherein the means for acquiring the instantaneous position of each mobile element comprise at least one camera (140) acquiring a video stream covering all or part of the field, and/or at least one beacon secured to said mobile element and at least one terminal for receiving a signal emitted by said beacon.

22. The system according to any one of claims 20 to 21, also comprising means for generating and displaying a map representing the dynamic occupation of the field.

23. The system according to any one of claims 20 to 22, also comprising means for recording the acquired data and for modifying by a user of all or part of the recorded data the instantaneous positions and/or speeds of the mobile elements.

FIG. 1

FIG. 2

3A

3B

FIG. 3

FIG. 4

5A

5B

FIG. 5

FIG. 6

FIG. 7

600

| 610 |

| 620 |

| 630 |

| 640 |

650

| 651 |

| 652 |

| 653 |

| 660 |

FIG. 8

FIG. 9

**EP 3 928 324 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 8279051 B
- WO 2017106390 A
- WO 03037464 A
- KR 20180063777